Europäisches Patentamt

European Patent Office

Office européen des brevets

(19)

(11) EP 1 512 415 A1

## (12) EUROPEAN PATENT APPLICATION
published in accordance with Art. 158(3) EPC

(43) Date of publication:
09.03.2005 Bulletin 2005/10

(21) Application number: 03728129.2

(22) Date of filing: 20.05.2003

(51) Int Cl.7: **A61K 48/00**, A61K 45/00,
A61K 31/7088, A61K 9/127,
A61P 1/16, A61P 13/02,
A61P 13/08, A61P 13/10,
A61P 13/12, A61P 19/02,
A61P 29/00

(86) International application number:
PCT/JP2003/006299

(87) International publication number:
WO 2003/099339 (04.12.2003 Gazette 2003/49)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 29.05.2002 JP 2002156524

(71) Applicant: Anges MG, Inc.
Toyonaka-shi, Osaka 560-0082 (JP)

(72) Inventors:
• TOMITA, Tetsuya c/o AnGes MG, Inc.
Toyonaka-shi, Osaka 560-0082 (JP)
• YOSHIKAWA, Hideki c/o AnGes MG, Inc.
Toyonaka-shi, Osaka 560-0082 (JP)
• MORISHITA, Ryuichi c/o AnGes MG, Inc.
Toyonaka-shi, Osaka 562-0082 (JP)

(74) Representative: Harding, Charles Thomas
D Young & Co
120 Holborn
London EC1N 2DY (GB)

## (54) DECOY COMPOSITION FOR TREATING AND PREVENTING INFLAMMATORY DISEASE

(57) It is intended to efficiently treat an inflammatory disease without causing side effects. This object can be achieved by providing a medicinal composition for treating and preventing an inflammatory disease and disorders, and disorders caused by the disease and disorders which contains at least one decoy of NF-κB or an analogous transcriptional factor and a pharmaceutically acceptable carrier; and a method of treating and preventing an inflammatory disease and disorders, and disorders caused by the disease and disorders which comprises the step of administering to a patient a composition containing at least one decoy of NF-κB or an analogous transcriptional factor and a pharmaceutically acceptable carrier.

Fig.2 Changes observed in the rat osteoarthritis models ( 7 weeks )

**Description**

TECHNICAL FIELD

The present invention is related to a composition comprising a compound (such as a nucleic acid and an analog thereof) specifically binding to a site where a transcriptional regulatory factor binds, and the use thereof. More specifically, the present invention is related to a composition comprising a decoy compound (such as nuclear factor κB (NF-κB) decoy) for treating inflammatory diseases or disorders.

BACKGROUND ART

A variety of diseases including asthma, cancers, heart diseases, aneurysms, autoimmune diseases, and viral infections manifest varying symptoms and signs and yet it has been suggested that an abnormal expression (an overexpression or underexpression) of one or a few proteins is a major etiologic factor in many cases. In general, the expression of those proteins is controlled by a variety of transcriptional regulatory factors such as transcription activating factors and transcription suppressing genes.

NF-κB is one of such transcriptional regulatory factors for genes encoding gene products important for inflammation and immune responses (Baeuerle PA. et al., Annu Rev Immunol. 1994; 12:141-79). NF-κB responds to various extracellular signals and migrates from the cytoplasm to the nucleus, and plays a pivotal role in the coordinated transactivation of several cytokines and adhesion molecule genes. Cooper et al. demonstrated a time-dependent increase in the DNA binding activity of NF-κB, which had a peak three days before rejection in an allogenic heart transplantation animal model (Cooper M. et al., Transplantation. 1998 Oct 15; 66(7):838-44). However, administration of PDTC, which is a potent inhibitor of NF-κB reduced the NF-κB activity peak in the model, significantly elongating the recipient animal survival.

The normal active form of human NF-κB is a heterodimer of two DNA binding subunits, 50 kDa subunit (p50) and 65kDa subunit (relAorp65) (Lenardo, Cell. 1989 Jul 28; 58 (2) :227-9; Libermann, Mol Cell Biol. 1990 May; 10 (5) : 2327-34; Satriano J, J Clin Invest. 1994 Oct; 94(4):1629-36; Neish AS et al., J Exp Med. 1992 Dec 1; 176(6):1583-93). In a cell which is not stimulated, NF-κB binds to an inhibition molecule known as IκB and hides within the cytoplasm. After a cell is stimulated, IκB is phosphorylated and then rapidly degraded. Thereafter, NF-κB is released from IκB, thereby making it possible for the transcription factor to translocate to the nucleus, in which the transcription factor binds to various DNA recognition sites to regulate gene expression (Baeurerle, 1994, supra). It has been suggested that the dissociation of the transcription factor NF-κB from the complex induces regulated transactivation of genes including interleukins (ILs)-1, -6, and -8; intracellular adhesion molecules; vascular cell adhesion molecules; and endothelial cell adhesion molecules, and plays a pivotal role in regulation of inflammatory changes (Lenardo, 1989 supra; Libermann, 1990 supra; Satriano J, 1994 supra; Neish, 1992 supra) . Therefore, blockade of NF-κB may attenuate gene-mediated cardiac ischemia-reperfusion.

NF-κB may be involved in the onset of progression of tumor malignancy (Rayet B et al., Oncogene 1999 Nov 22; 18(49)6938-47); NF-κB is involved in responses of tumor cells to hypoxia stress (Royds JA et al., Mol Pathol 1998 Apr; 51(2):55-61); NF-κB inhibits expression of cytokines and adhesion molecules in synovial membrane cells derived from chronic rheumatoid arthritis patients (Tomita T et al., Rheumatology (Oxford) 2000 Jul; 39(7):749-57); suppression of coordination between a plurality of transcription factors including NF-κB changes the malignant phenotypes of various tumors (Denhardt DT, Crit Rev Oncog 1996; 7(3-4):261-91); downregulation of NF-κB activation due to green tea polyphenol blocks induction of nitric oxide synthesizing enzyme, and suppresses A431 human epidermoid carcinoma cells (Lin JK et al., Biochem Pharmacol 1999 Sep 15; 58(6):911-5); amyloid β peptide observed in the brains of Alzheimer's disease patients binds to 75-kD neurotrophic receptor (p75NTR) in neuroblastoma cells to activate NF-κB in a time-dependent manner and a dose-dependent manner (Kuper P et al., J Neurosci Res 1998 Dec 15; 54(6):798-804) ; TNF-α plays an important role in the onset of glomerulonephritis (Ardaillou et al., Bull Acad Natl Med 1995 Jan; 179 (1)103-15).

A transcription factor decoy for NF-κB inhibits expression of cytokines and adhesion molecules in vivo in murine nephritis induced by TNF-α (Tomita N et al., Gene Ther 2000 Aug; 7(15)1326-32); and the like.

It was suggested that NF-κB suppresses MMP1 and MMP9, members of matrix metalloproteinase (MMP), at the transcription level (Eberhardt W, Huwiler A, Beck KF, Walpen S, Pfeilschifter J. J Immunol 2000 Nov 15, 165(10), 5788-97; M, Baker AH, Newby AC. Biochem Biophys Res Commun. Bond 1999 Oct 22, 264 (2), 561-7; Bond M, Fabunmi RP, Baker AH, Newby AC. FEBS Lett 1998 Sep 11, 435 (1), 29-34; and Kim H, Koh G. Biochem Biophys Res Commun. 2000 Mar 16, 269(2), 401-5). MMP is a polygene family of zinc-dependent enzymes involved in degradation of extracellular matrix components.

MMP plays an important role in invasion of cancer cells by mediating degradation of extracellular matrix protein. A number of studies suggested the involvement of MMP and MMP inhibitors (TIMP) in the progression of cancer:

the TIMP1 level in serum may be used as a marker for prognosis and diagnosis of colon and rectum cancer, and a selective marker for metastatic cancer (Pellegrinl P et al., Cancer Immunol Immunother 2000 Sep; 49 (7) :388-94) ; expression and activity of MMP2 and MMP9 in human urinary bladder cancer cells are affected by tumor necrosis factor $\alpha$ and $\gamma$ interferon (Shin KY et al., Cancer Lett 2000 Oct 31; 159(2) :127-134); MMP2, MMP9 and MT1-MMP, and their inhibitors, TIMP1 and TIMP2, are expressed in an ovarian epithelium tumor (Sakata K et al., Int J Oncol 2000 Oct; 17(4):673-681); the level of each of MMP1, MMP2, MMP3 and MMP9 and the overall MMP activity are upregulated in colon and rectum tumors, and MMP1 is most important for progression of colon and rectum cancer (Baker EA et al., Br J Surg 2000 Sep; 87(9):1215-1221) ; activated MMP2 plays an important role in invasion by urothelial cancer, and also the expression level of the activated MMP2 can be used as a useful prognosis index (Kaneda K et al., BJU Int 2000 Sep; 86(4): 53-557); a prostaglandin synthesis inhibitor inhibits invasion of human prostate tumor cells, and reduces the release of MMP (Attiga FA et al., Cancer Res 2000 Aug 15; 60(16):4629-37) ; the MMP activity of a serum euglobulin fraction increases in breast cancer and lung cancer patients, and may be used as a tumor marker for these cancers (Farias E et al., Int J Cancer 2000 Jul 20; 89(4):389-94) ; a MMPinhibitor inhibits gelatin-degrading activity in tumor cells (Ikeda M et al., Clin Cancer Res 2000 Aug; 6(8): 3290-6) ; induction of MMP9 due to a membrane protein LMP1 contributes to metastatic of nasopharyngeal cancer (NPC) (Horikawa T et al., Caner 2000 Aug 15; 89(4):715-23); MMP plays an important role in an early stage of angioplasty, and a MMP inhibitor suppresses invasion and morphogenesis of human microvascular endothelial cells (Jia MC et al., Adv Exp Med Biol 2000; 476:181-94) ; MMP9 is expressed in invasive and recurrent pituitary adenoma and hypophysis cancer (Turner HE et al., J Clin Endocrinol Metab 2000 Aug; 85(8):2931-5) ; and the like.

MMP is also known to be involved in development of aortic aneurysm: MMP is involved in formation and rupture of cerebral aneurysm (Gaetani P et al., Neurol Res 1999 Jun; 21(4):385-90); a MMP-9 promotor is a risk factor for cerebral aneurysm (Peters DG et al., Stroke 1999 Dec; 30(12):2612-6); inhibition of MMP inhibits the growth of microaneurysm in an aneurysm model (Treharne GD et al., Br J Surg 1999 Aug; 86(8):1053-8); and the like. MMP is secreted from migrating vascular smooth muscle cells, macrophage, and the like, and destroys collagen, elastin, and the like present in blood vessel walls, whereby the tension of the blood vessel is lost and the blood vessel does not resist the blood pressure and its diameter is expanded. In fact, in the blood vessel of an aneurysm, significant destruction of elastin is observed.

According to data obtained by measuring the aorta diameter of from 35-year-old to 80-year old adult males, the average was 1.5 cm to 2.0 cm. In general, the aorta having a diameter beyond 1.5 times as great as the average value is judged as an aortic aneurysm. However, according to the above-described data, one in every 400 people had an aneurysm having a diameter of 3 cm or more which is judged as aortic aneurysm. Therefore, although the degree of risk of aorta rupture is not considered here, the prevalence of aortic aneurysm is relatively high in 35-year-old to 80-year old adult males. The prevalence is believed to be even greater in males aged 65 and above.

It has been reported that a MMP inhibitor suppresses the expansion of blood vessel diameter in an aortic aneurysm model in rat abdomen. A MMP inhibitor may be used in therapy for glomerulonephritis (Marti HP, Schweiz Med Wochenschr 2000 May 27; 130 (21) ; 784-8) . However, systemic administration of a MMP inhibitor causes severe side effects, and has difficulty in clinical applications for treatment (therapy and prevention) of various diseases.

Synthetic ODN as a "decoy compound" cis-element blocks a nuclear factor from binding to the promoter region of its intended gene, thereby inhibiting gene transactivation of in vitro and in vivo assay systems (Sullenger, J Virol. 1991 Dec; 65(12):6811-6; Morishita R. et al., Contrib Nephrol. 1996; 118:254-64). Such a decoy strategy has been proposed for treatment of certain human diseases. The present inventors previously reported that transfection of E2F decoy ODN as a gene therapy model for restenosis inhibited neointimal proliferation after balloon-injury (Morishita, Proc Natl Acad Sci U S A. 1995 Jun 20; 92(13):5855-9). Recently, the present inventors succeeded in *in vivo* protection of myocardiac muscle from ischemic injury using a decoy for NF-κB in rats.

(Problems to be solved by the invention)

Thus, it has been suggested that NF-κB is involved in various diseases via expression of a number of genes under the transcription control thereof. However, no method for effectively treating a disorder or disease associated with inflammatory diseases, particularly a non-invasive treatment method, has been provided. Particularly, as described above, inflammatory diseases such as rheumatoid arthritis and osteoarthritis and the like are not rare diseases. As society ages, an increase in arteriosclerotic diseases inevitably leads to an increase in aortic aneurysm diseases. Considering the aging of patients, it is ideal to suppress directly the growth of inflammatory diseases using a pharmaceutical agent, however, to date such a means is not available. There is a desperate demand for development of a low-invasive therapy and prevention method for inflammatory diseases.

Inflammatory diseases refer to any diseases or disorders associated with inflammation. In particular, effective treatment against diseases with inflammation in joint sites (for example, rheumatoid arthritis, osteoarthritis, scapulohumeral periarthritis and the like) with no side effects or significantly reduced side effects, are demanded.

SUMMARY OF INVENTION

The present invention provides a composition for treating or preventing an inflammatory disease or disorder as well as disorders due to the disease and disorder, comprising at least one NF-κB decoy or a decoy of a similar transcriptional factor, and a pharmaceutically acceptable carrier. The present invention further provides a method for treating or preventing an inflammatory disease or disorder as well as disorders due to the disease and disorder, comprising the step of administrating to a subject a composition comprising at least one NF-κB decoy or a decoy of a similar transcriptional factor, and a pharmaceutically acceptable carrier.

In an embodiment, inflammatory diseases include a variety of inflammation such as nephritis, hepatitis, arthritis (for example, rheumatoid arthritis, osteoarthritis or the like), acute renal failure, chronic renal failure, arteriosclerosis, glomerulonephritis, pyelonephritis, urocystitis, prostatitis, urethritis, epididymitis, testitis and the like. The disorders are those associated with the above-listed inflammatory diseases. The pharmaceutically acceptable carrier may be a liposome. The NF-κB decoy may comprise a sequence of GGATTTCCC. In one embodiment, the decoy or composition may be directly delivered to the joint.

Accordingly, the present invention provides the following:

1. A pharmaceutical composition for treating and preventing an inflammatory disease or disorder, comprising:

at least one NF-κB decoy, and
a pharmaceutically acceptable carrier.

2. The composition according to Item 1, wherein said disease or disorder is at least one selected from the group consisting of nephritis, hepatitis, arthritis, acute renal failure, chronic renal failure, arteriosclerosis, glomerulonephritis, pyelonephritis, urocystitis, prostatitis, urethritis, epididymitis and testitis.

3. The composition according to Item 1, wherein the pharmaceutically acceptable carrier is a liposome.

4. The composition according to Item 1, wherein the NF-κB decoy comprises a sequence of GGATTTCCC.

5. The composition according to Item 1, wherein the disease or disorder is osteoarthritis or rheumatoid arthritis.

6. The composition according to Item 1, which is adapted for administration to a knee joint.

7. The composition according to Item 6, wherein the NF-κB decoy comprises at least 10 mg.

8. The composition according to Item 6, wherein the NF-κB decoy comprises at least 30 mg.

9. A pharmaceutical composition for treating and preventing an articular disease or disorder, comprising:

at least one NF-κB decoy, and
a pharmaceutically acceptable carrier.

10. A composition according to Item 9, wherein said disease or disorder is at least one selected from the group consisting of rheumatoid arthritis, osteoarthritis, periarthritis humeroscapularis, cervicobrachial syndrome and secondary arthritis.

11. A composition according to Item 9, wherein said pharmaceutically acceptable carrier is a liposome.

12. A composition according to Item 9, wherein said NF-κB decoy comprises a sequence of GGATTTCCC.

13. A composition according to Item 9, wherein said disease or disorder is osteoarthritis or rheumatoid arthritis.

14. A composition according to Item 9, wherein said disease or disorder is rheumatoid arthritis, and said decoy comprises at least a therapeutically effective amount.

15. A composition according to Item 14, wherein said therapeutically effective amount is at least 0.01 mg/kg bodily weight.

16. A composition according to Item 9, which is adapted to administration into a knee joint.

17. A composition according to Item 16, wherein said NF-κB decoy comprises at least 10mg.

18. A composition according to Item 16, wherein said NF-κB decoy comprises at least 30mg.

19. A method for treating or preventing inflammatory disease or disorder, comprising:

administering to a patient a composition comprising:

at least one NF-κB decoy; and
a pharmaceutically acceptable carrier.

20. A method according to Item 19, wherein said disease or disorder is at least one selected from the group consisting of nephritis, hepatitis, arthritis, acute renal failure, chronic renal failure, arteriosclerosis, glomerulonephritis, pyelonephritis, urocystitis, prostatitis, urethritis, epididymitis and testitis.

21. A method according to Item 19, wherein the pharmaceutically acceptable carrier is a liposome.

22. A method according to Item 19, wherein the NF-κB decoy comprises a sequence of GGATTTCCC.

23. A method according to Item 19, wherein the disease or disorder is osteoarthritis or rheumatoid arthritis.

24. A method according to Item 19, wherein said composition is adapted for administration to a knee joint.

25. A method according to Item 24, wherein said composition comprises at least 10 mg of the NF-κB decoy.

26. A method according to Item 24, wherein said composition comprises at least 30 mg of the NF-κB decoy.

27. A method for treating and preventing articular disease or disorder, comprising:

administering to a patient a composition comprising:

at least one NF-κB decoy; and
a pharmaceutically acceptable carrier.

28. A method according to Item 27, wherein said disease or disorder is at least one selected from the group consisting of rheumatoid arthritis, osteoarthritis, periarthritis humeroscapularis, cervicobrachial syndrome and secondary arthritis.

29. A method according to Item27, wherein saidpharmaceutically acceptable carrier is a liposome.

30. A method according to Item 27, wherein said NF-κB decoy comprises a sequence of GGATTTCCC.

31. A method according to Item 27, wherein said disease or disorder is osteoarthritis or rheumatoid arthritis.

32. A method according to Item 27, wherein said disease or disorder is rheumatoid arthritis, and said decoy comprises at least a therapeutically effective amount.

33. A method according to Item 27, wherein said therapeutically effective amount is at least 0.01 mg/kg bodily weight.

34. A method according to Item 27, wherein said composition is adapted for administration to a knee joint.

35. A method according to Item 34, wherein said composition comprises at least 10 mg of said NF-κB decoy.

36. A method according to Item 34, wherein said composition comprises at least 30 mg of said NF-κB decoy.

37. Use of at least one NF-κB decoy in manufacturing a medicament for treating and preventing an inflammatory disease or disorder.

38. Use according to Item 37, wherein said disease or disorder is at least one selected from the group consisting of nephritis, hepatitis, arthritis, acute renal failure, chronic renal failure, arteriosclerosis, glomerulonephritis, pyelonephritis, urocystitis, prostatitis, urethritis, epididymitis and testitis.

39. Use according to Item 37, wherein the pharmaceutically acceptable carrier is a liposome.

40. Use according to Item 37, wherein the NF-κB decoy comprises a sequence of GGATTTCCC.

41. Use according to Item 37, wherein the disease or disorder is osteoarthritis or rheumatoid arthritis.

42. Use according to Item 37, wherein said composition is adapted for administration to a knee joint.

43. Use according to Item 42, wherein said composition comprises at least 10 mg of the NF-κB decoy.

44. Use according to Item 42, wherein said composition comprises at least 30 mg of the NF-κB decoy.

45. Use of at least one NF-κB decoy in manufacturing a medicament for treating and preventing an articular disease or disorder.

46. Use according to Item 45, wherein said disease or disorder is at least one selected from the group consisting of rheumatoid arthritis, osteoarthritis, periarthritis humeroscapularis, cervicobrachial syndrome and secondary arthritis.

47. Use according to Item 45, wherein said pharmaceutically acceptable carrier is a liposome.

48. Use according to Item 45, wherein said NF-κB decoy comprises a sequence of GGATTTCCC.

49. Use according to Item 45, wherein said disease or disorder is osteoarthritis or rheumatoid arthritis.

50. Use according to Item 45, wherein said disease or disorder is rheumatoid arthritis, and said decoy comprises at least a therapeutically effective amount.

51. Use according to Item 45, wherein said therapeutically effective amount is at least 0.01 mg/kg bodily weight.

52. Use according to Item 45, wherein said composition is adapted for administration to a knee joint.

53. Use according to Item 52, wherein said composition comprises at least 10 mg of said NF-κB decoy.

54. Use according to Item 52, wherein said composition comprises at least 30 mg of said NF-κB decoy.

These and other advantages of the present invention will be apparent to those skilled in the art upon reading and understanding the following detailed description, referring to the appended drawings if necessary.

BRIEF DESCRIPTION OF THE DRAWINGS

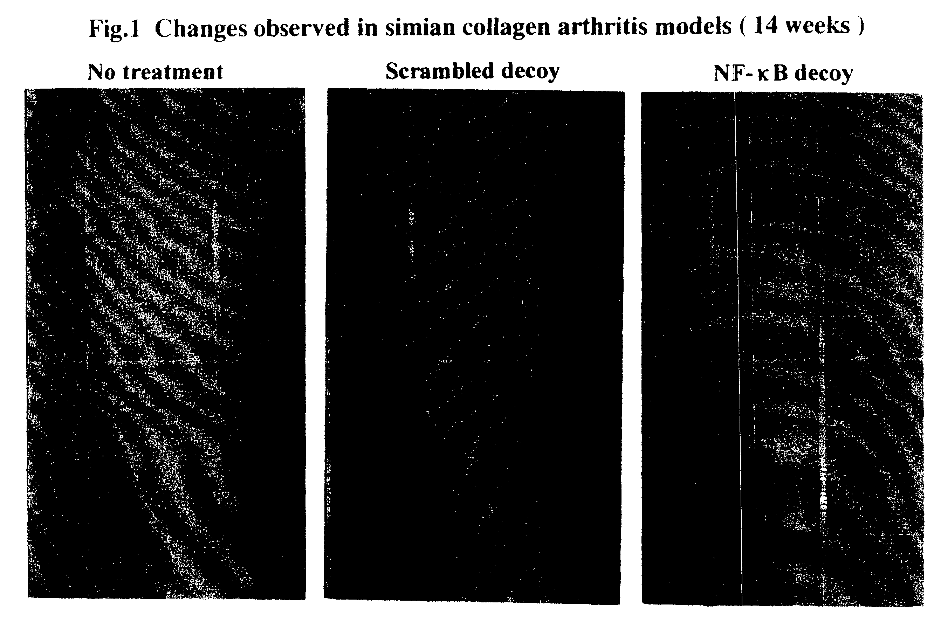

Figure **1** is a radiograph showing appearance of the change by the decoy of the present invention in the joint of simian collagen articulitis model. Models having received no treatment and scrambled decoy have no effects, whereas the model having received the treatment of the decoy of the present invention showed a number significant improvements such as change in synovium inflammation, suppression of articular swelling, and suppression of osteoclasis in the articular cartilage and joint, and the like.

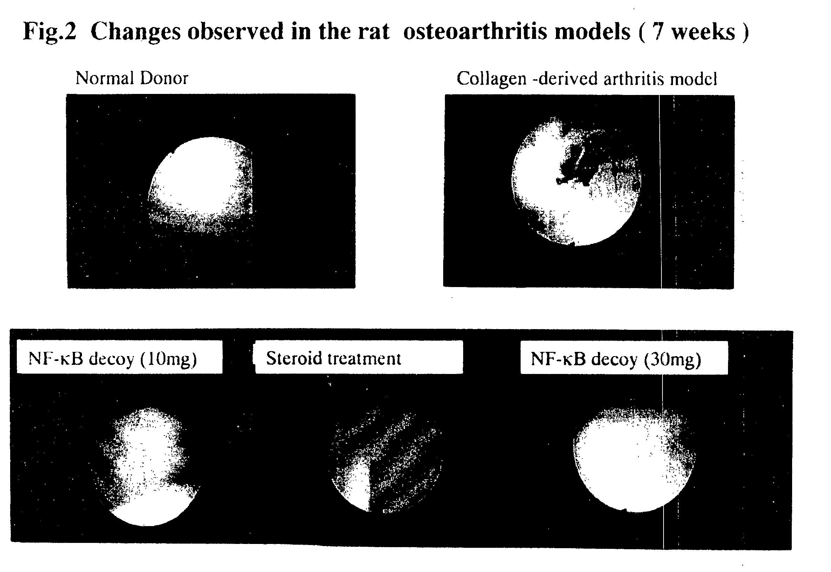

Figure **2** shows a photograph of a section slide of the joint showing the effects of an NF-κB against the osteoarthritis rat model. It is shown that a normal donor, a collagen-derived articulitis model, and effects of NF-κB decoys (10mg and 30 mg administration), andasteroidagainstthemodel. Administration of ten mg of the decoy already attained significant effects and administration of 30 mg of decoy destroyed the articulitis. These effects are beyond those attained by the steroid. In addition, contrary to the steroid treatment, there was no side effect.

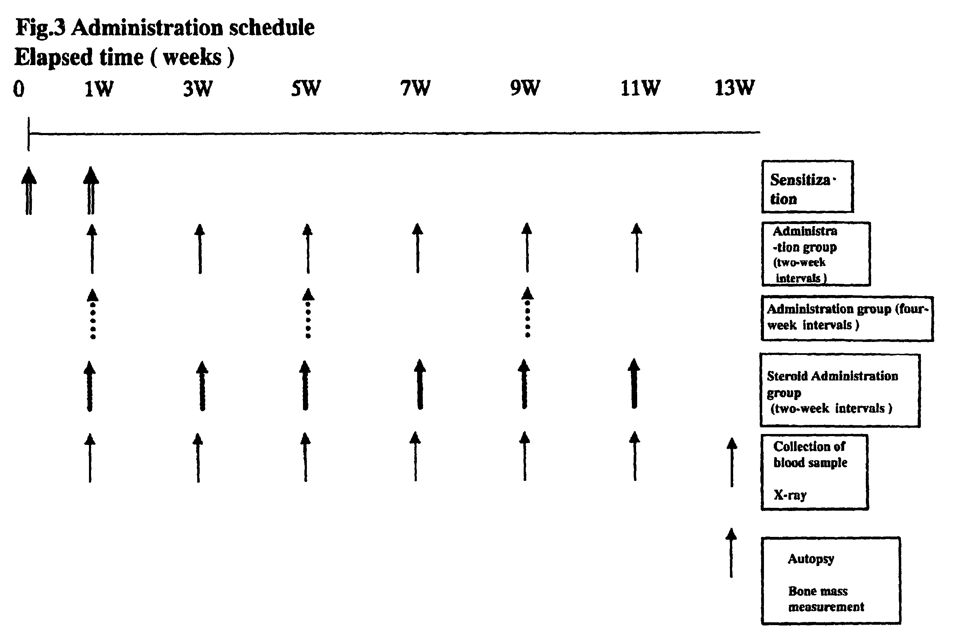

Figure **3** shows a regimen for treatment of collagen derived articulitis according to the present invention.

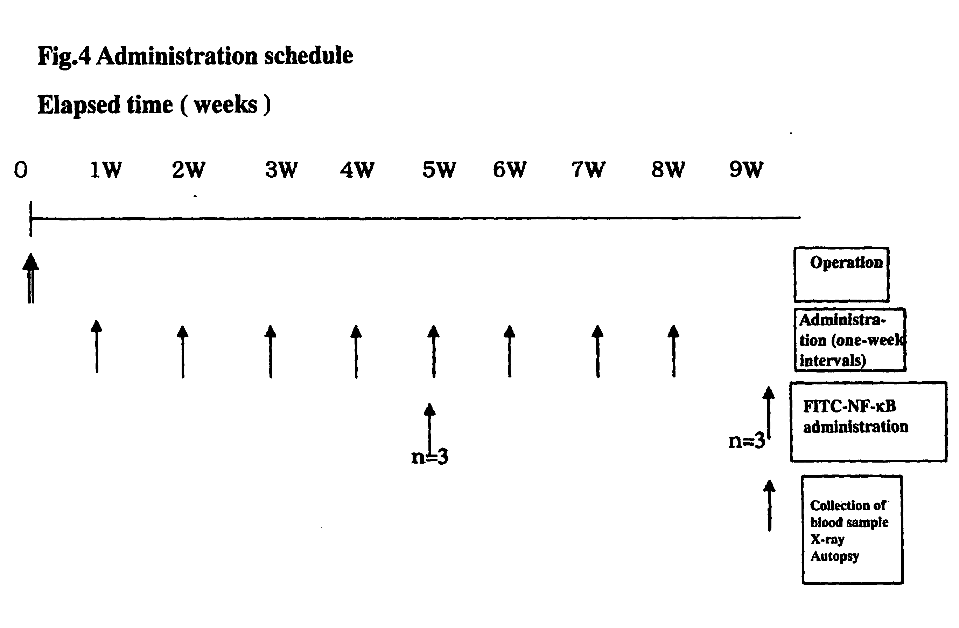

Figure **4** shows a regimen for treatment of osteoarthritis according to the present invention.

BEST MODE FOR CARRYING OUT THE INVENTION

Hereinafter, the present invention will be described. It should be understood throughout the present specification that articles for a singular form (e.g., "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise mentioned. It should also be understood that the terms as used herein have definitions typically used in the art unless otherwise mentioned. Accordingly, unless otherwise defined, all technical and scientific terms used herein shall have the same meaning as generally understood by those skilled in the art to which the present invention pertains. If there is any inconsistency, the present specification precedes including definitions.

As used herein the term "decoy" or "decoy compound" refers to a compound which mimics a chromosomal site to which a transcriptional factor such as NF-κB binds, or which mimics a chromosomal site of a gene controlled by a transcriptional factor such as NF-κB and the like, to which the transcriptional regulatory factor binds (hereinafter referred to "target binding site"), thereby competing with the chromosomal binding site for binding to the transcriptional factor. Typically, the decoy or the decoy compound includes a nucleic acid and analogs thereof.

When a decoy is present within a nucleus, the decoy competes with a transcriptional regulatory factor for a target binding site for the transcriptional regulatory factor. As a result, a biological function which would be generated by binding of the transcriptional regulatory factor to the target binding site is inhibited. The decoy contains at least one nucleic acid sequence capable of binding to a target binding sequence. A decoy can be used for preparation of a pharmaceutical composition according to the present invention as long as the decoy has activity to bind to a target binding sequence.

Examples of the decoy include oligonucleotides containing GGGATTTC concerning NF-κB. Preferable examples of the decoy include 5'-CCTTGAAGGGATTTCCCTCC-3' (SEQ ID NO: 1) (NF-κB decoy) , 5'-GATCTAGGGATTTCCGGGAAATGAAGCT-3' (SEQ IDNO: 2) (STAT-1 decoy), 5'-AGCTTGAGATAGAGCT-3' (SEQ ID NO: 3) (GATA-3 decoy), 5'-GATCAAGACCTTTTCCCAAGAAATCTAT-3' (SEQ ID NO: 4) (STAT-6 decoy), 5'-AGCTTGTGAGTCAGAAGCT-3' (SEQ ID NO: 5) (AP-1 decoy), and 5'-AATTCACCGGAAGTATTCGA-3' (SEQ IDNO: 6) (Ets decoy) , 5'-TGACGTCA-3' (CRE decoy sequence) or oligonucleotide containing complements thereof, mutants thereof, or compounds containing these molecules therein. The oligonucleotides may be either DNA or RNA. The oligonucleotides may also include a modified nucleic acid and/or pseudonucleic acid therein. Further, these oligonucleotides may be mutants thereof, or compounds containing them therein. The oligonucleotides may have a single strand or double strands, ormaybelinearorcircular. Themutants are nucleic acids having the above-described sequences, a part of which has a mutation, a substitution, an insertion, or a deletion, and which specifically antagonize a transcription factor, such as NF-κB and the like, or another transcriptional regulatory factors for a gene controlled by a transcription factor, such as NF-κB and the like, with respect to the nucleic acid binding site to which the factor binds. More. preferable examples of the decoy for the transcription factor, such as NF-κB and the like, or the other transcriptional regulatory factor for a gene controlled by a transcription factor, such as NF-κB and the like, include double-strand oligonucleotides containing one or a plurality of the above-described nucleic acid sequences, or mutants thereof. Nucleic acids containing one or a plurality of the above-described nucleic acid sequences are called a double decoy when the number of nucleic acid sequences contained is two or a triple decoy, when the number of nucleic acid sequences contained is three, indicating the number of nucleic acid sequences.

The oligonucleotides for use in the present invention include oligonucleotides modified so as to resist in vivo degradation, and the like, such as oligonucleotides (S-oligo) having a thiophosphatediester bond which is a phosphatediester bond whose oxygen atom is replaced with a sulfur atom, oligonucleotides whose phosphatediester bond is substituted with a methylphosphate group having no electronic charge, and the like.

The decoy of the present invention can be produced with chemical or biochemical synthesis methods known in the art. For example, when a nucleic acid is used as a decoy compound, nucleic acid synthesis methods commonly used in genetic engineering can be employed. For example, a DNA synthesizer may be used to directly synthesize intended decoy nucleic acids. Further, these nucleic acids, nucleic acids containing the nucleic acids, or parts thereof may be synthesized, followed by amplification using a PCR method, a cloning vector, and the like. Furthermore, nucleic acids obtained by these methods are cleaved using a restriction enzyme, or the like, and linked or the like using DNA ligase, or the like to produce an intended nucleic acid. To obtain decoy nucleic acids which are more stable in cells, base, sugar and phosphate portions of the nucleic acids may be subjected to chemical modification, such as alkylation, acylation, or the like.

The present invention provides a pharmaceutical composition comprising the above-described decoy compound alone or in combination with a stabilizing compound, a diluent, a carrier or another component, or a pharmaceutical agent.

The pharmaceutical composition of the present invention may be used in such a form that the decoy is taken into cells in an affected part or cells in an intended tissue.

The pharmaceutical composition of the present invention is administered in any aseptic biocompatible pharmaceutical carrier (including, but not limited to, physiological saline, buffered physiological saline, dextrose, and water). A pharmaceutical composition of any of these molecules mixed with an appropriate excipient, an adjuvant, and/or a pharmaceutically acceptable carrier may be administered to patients alone or in combination with another pharmaceutical agent in a pharmaceutical composition. In an embodiment of the present invention, the pharmaceutically acceptable carrier is pharmaceutically inactive.

The administration of the pharmaceutical composition of the present invention is achieved orally or parenterally. Parenteral delivery methods include topical, intra-arterial (e.g., through a carotid artery), intramuscular, subcutaneous, intramedullary, into subarachnoid space, intraventricular, intravenous, intraperitoneal, or intranasal administrations. In the present invention, any route may be possible as long as the composition is delivered through the route to a site to be treated, particularly, inflammatory sites. Therefore, the present invention provides a novel treatment method for carrying out gene transfection to the inflammatory sites or artery and a composition therefor.

In addition to the decoy compound, these pharmaceutical compositions contain an appropriate pharmaceutically acceptable carrier, including another compound for accelerating the processing of the decoy compound so as to produce an excipient or a preparation which can be pharmaceutically used. Further details of techniques for preparation and administration of the decoy compound are described in, for example, the latest version of Japanese Pharmacopoeia with the latest supplement, and "REMINGTON'S PHARMACEUTICAL SCIENCES" (Maack Publishing Co., Easton, PA).

A pharmaceutical composition for oral administration may be prepared using a pharmaceutically acceptable carrier well known in the art in an administration form suitable for administration. Such a carrier can be prepared as a tablet, a pill, a sugar-coated agent, a capsule, a liquid, a gel, a syrup, a slurry, a suspension, or the like, which is suited for the patient to take the pharmaceutical composition.

The pharmaceutical composition for oral use may be obtained in the following manner: an active compound is combined with a solid excipient, the resultant mixture is pulverized if necessary, an appropriate compound is further added if necessary to obtain a tablet or the core of a sugar-coated agent, and the granular mixture is processed. The appropriate excipient may be a carbohydrate or protein filler, including, but not being limited to, the following: sugar including lactose, sucrose, mannitol, or sorbitol; starch derived from maize, wheat, rice, potato, or other plants; cellulose such as methylcellulose, hydroxypropylmethylcellulose, or sodium carboxymethylcellulose; and gum including gum Arabic and gum tragacanth; and protein such as gelatin and collagen. A disintegrant or a solubili zing agent such as crosslinked polyvinyl pyrrolidone, agar, alginic acid or a salt thereof (e.g., sodium alginate) may be used if necessary.

The sugar-coated agent core is provided along with an appropriate coating, such as a condensed sugar solution. The sugar-coated agent core may also contain gum arabic, talc, polyvinyl pyrrolidone, carbopolygel, polyethylene glycol, and/or titanium dioxide, a lacquer solution, and an appropriate organic solvent or a solvent mixed solution. To identify a product, or characterize the amount of an active compound (i.e., dose), dye or pigment may be added to tablets or sugar-coated agents.

The pharmaceutical preparation which may be orally used may contain, for example, a soft sealed capsule consisting of a gelatin capsule, gelatin and coating (e.g., glycerol or sorbitol). The gelatin capsule may contain an active component mixedwith a filler or binder such as lactose or starch, a lubricant such as talc or magnesium stearate, and optionally a stabilizer. In the soft capsule, the decoy compound may be dissolved or suspended in an appropriate liquid, such as fatty oil, liquid paraffin or liquid polyethylene glycol, with or without a stabilizer.

The pharmaceutical preparation for parenteral administration contains an aqueous solution of an active compound. For the purpose of injection, the pharmaceutical composition of the present invention is prepared in an aqueous solution, preferably Hank's solution, Ringer's solution, or a physiologically suitable buffer such as a buffered physiological saline. The aqueous suspension for injection may contain a substance for increasing the viscosity of a suspension (e.g., sodium carboxymethylcellulose, sorbitol, or dextran). Further, the suspension of the active compound may be prepared as an appropriate oily suspension. Appropriate lipophilic solvents or vehicles include a fatty acid such as sesame oil, synthetic fatty acid ester such as ethyl oleate or triglyceride, or liposomes. The suspension may contain a stabilizer which allows a high-concentration solution preparation, or an appropriate pharmaceutical agent or reagent for increasing the solubility of the compound, if necessary.

For topical or intranasal administration, an appropriate penetrant for the specific barrier to be penetrated may be used in the preparation. Such a penetrant is generally known in the art.

The pharmaceutical composition of the present invention may be produced using a method similar to methods known in the art (e.g., conventional mixing, dissolution, rendering to granules, preparation of a sugar-coated agent, elutriation, emulsification, capsulation, inclusion, freeze drying or lyophilization).

Preferably, in the case of parenteral administration, such as topical administration or infusion from a cervical portion to cell of an affected part or cells of an intended tissue, the pharmaceutical composition of the present invention may contain a synthetic or naturally-occurring hydrophilic polymer as a carrier. Examples of such a hydrophilic polymer include hydroxypropylcellulose and polyethylene glycol. The decoy compound of the present invention may be mixed with the above-described hydrophilic polymer in an appropriate solvent. The solvent may be removed by a method such as air drying. The resultant compound may be shaped into a desired form, such as a sheet, and then may be given to a target site. Such a preparation containing a hydrophilic polymer has a small moisture content, and an excellent shelf life, and an excellent retentivity of the decoy, a compound, since the preparation absorbs water to be turned into gel when used.

Alternatively, when a nucleic acid or a modification thereof is employed as a decoy, the pharmaceutical composition of the present invention is advantageously used in a form which is generally used in gene introduction methods, such as a membrane fusion liposome preparation using Sendai virus (HVJ) or the like, a liposome preparation using endocytosis or the like, a preparation containing a cationic lipid such as Lipofectamine (Gibco BRL) or the like, or a viral preparation using a retrovirus vector, an adenovirus vector, or the like. Particularly, a membrane fusion liposome preparation is preferable.

The liposome preparation is any of the liposome constructs which are a large unilamellar vesicle (LUV), a multilammelarvesicle (MLV) , and a small unilamellar vesicle (SUV). The LUV has a particle size ranging from about 200 to about 1000 nm. The MLV has a particle size ranging from about 400 to about 3500 nm. The SUV has a particle size ranging from about 20 to about 50 nm. The membrane fusion liposome preparation using HVJ or the like preferably employs MLV having a particle size ranging from 200 nm to 1000 nm.

There is no particular limitation on a method for producing liposomes as long as the liposomes hold a decoy. The liposomes can be produced by a commonly used method, such as, for example, a reversed phase evaporation method (Szoka, F et al., Biochim. Biophys. Acta, Vol. 601 559(1980)), an ether infusion method (Deamer, D.W.: Ann. N.Y. Acad. Sci., Vol. 308 250(1978)), a surfactant method (Brunner, J et al.: Biochim. Biophys. Acta, Vol. 455 322 (1976)), or the like.

Examples of lipids for forming a structure of a liposome include phospholipids, cholesterol, nitrogen lipids, and the like. Generally, phospholipids are preferable, including naturally-occurring phospholipids, such as phosphatidylcholine, phosphatidylserine, phosphatidylglycerol, phosphatidylinositol, phosphatidylethanolamine, phosphatidic acid, cardiolipin, sphingomyelin, egg yolk lecithin, soybean lecithin, lysolecithin, and the like, or the corresponding phospholipids hydrogenated by a commonly used method, and in addition, synthetic phospholipids, such as dicetylphosphate, distearoylphosphatidylcholine, dipalmitoylphosphatidylcholine, dipalmitoylphosphatidylethanolamine, dipalmitoylphosphatidylserine, eleostearoylphosphatidylcholine, eleostearoylphosphatidylethanolamine, eleostearoylphosphatidylserine, and the like.

The lipids including these phospholipids can be used alone or with at least two in a combination. In this case, lipids having an atom group having a positive group, such as ethanolamine, choline, or the like, within the molecule can be used to increase the binding rate of an electrically negative decoy nucleic acid. In addition to the major phospholipids used to form liposomes, an additive, such as cholesterols, stearylamine, $\alpha$-tocopherol, or the like, which are generally known as an additive for formation of liposomes, can be used.

The thus-obtained liposomes can additionally contain a substance for promoting membrane fusion, such as a membrane fusion promoting protein purified from HVJ, inactivated HVJ, Sendai virus, or the like, so as to accelerate uptake into cells at an affected site or cells in an intended tissue.

An exemplary method for producing a liposome preparation will be specifically described below. For example, the above-described substance for forming a liposome is dissolved along with cholesterol in an organic solvent, such as tetrahydrofuran, chloroform, ethanol, or the like. The resultant solution is put into an appropriate vessel, followed by removal of the solvent under reduced pressure, thereby forming a film of the liposome forming substance on an inside wall of the vessel. A buffer solution containing a decoy is added to the vessel followed by agitation. The above-described membrane fusion promoting substance is added to the resultant liposome if necessary, followed by isolation of the liposome. The thus-obtained liposome containing the decoy can be suspended in an appropriate solvent or can be freeze-dried and thereafter dispersed in an appropriate solvent. The resultant suspension can be used in treatment. The membrane fusion promoting substance may be added in the interim period after the isolation of the liposome and before use.

The composition or kit of the present invention may further comprise a biocompatible material. Such a biocompatible material may contain at least one selected from the group consisting of silicone, collagen, gelatin, glycolic acid-lactic acid copolymers, ethylene-vinyl acetate copolymers, polyurethane, polyethylene, polytetrafluoroethylene, polypropylene, polyacrylate, andpolymethacrylate, for example. Silicone is preferable because of its ease of molding. Examples of biodegradable polymers include collagen; gelatin; polymers or copolymers synthesized by dehydration polycondensation without a catalyst from at least one selected from the group consisting of α-hydroxycarboxylic acids (e.g., glycolic acid, lactic acid, hydroxybutyric acid, and the like), hydroxydicarboxylic acids (e.g., malic acid and the like) and hydroxytricarboxylic acids (e.g., citric acid and the like), or a mixture thereof; poly-α-cyanoacrylate ester; polyamino acids (e.g., poly-γ-benzil-L-glutamic acid and the like) , polymerizable acid anhydrides of maleic anhydride-based copolymers (e.g., styrene-maleic acid copolymers and the like) ; and the like. The type of the polymerization is any of random, block, and graft. When α-hydroxycarboxylic acids, hydroxydicarboxylic acids, and hydroxytricarboxylic acids have the center of optical activity in the molecule, any of D-isomers, L-isomers, and DL-isomers can be used. Preferably, a glycolic acid-lactic acid copolymer may be used.

In one embodiment, the composition or kit of the present invention may be provided in a form of sustained release. The sustained-release dosage form may be any known form in the art as long as it is used in the present invention. Examples of such a form include rod forms (pellet forms, cylinder forms, needle forms, and the like) , tablet forms, disk forms, ball forms, and sheet forms. Methods for preparing a sustained-release form are known in the art and disclosed in, for example, Japanese Pharmacopoeia, U.S. Pharmacopoeia, other countries' Pharmacopoeias, and the like. Examples of methods for producing a sustained-release preparation (prolonged-administration preparation) include a method of utilizing disaggregation of a drug from a complex, a method of using an aqueous suspension for injection, a method of using an oil solution for injection or an oil suspension for injection, a method of using an emulsion for injection (o/w type and w/o type emulsions for injection, and the like), and the like.

In the case of the sustained-release form, a sustained-release preparation (mini-pellet preparation or the like) can be embedded in the vicinity of a site to which the preparation is to be administered. Alternatively, an osmotic pump or the like can be used to administer the sustained-release preparation continuously and gradually.

Injection agents can be prepared by a method well known in the art. For example, a component is dissolved in an appropriate solvent (physiological saline, a buffer solution such as PBS, sterilized water, or the like), followed by filter sterilization using a filter or the like. Thereafter, an aseptic vessel (e.g., ampoule or the like) is filled with the resultant solution, thereby preparing the injection agent. The injection agents may contain a commonly used pharmaceutical carrier if necessary. In the case of the liposome form, a reagent required for liposome preparations, such as suspension agents, cryogen, and cryogen condensed by centrifugation, can be added. The liposome is preferably administered parenterally. Therefore, when the liposome is administered, a non-invasive catheter, a non-invasive syringe, or the like can be used for the administration. As an administration method usinganon-invasive catheter, the composition of the present invention is infused directly into brain or through a carotid artery, for example.

The pharmaceutical composition of the present invention includes a composition containing an effective amount of decoy compound which can achieve the intended purpose of the decoy compound. "Therapeutically effective amount" or "pharmacologically effective amount" are terms which are well recognized by those skilled in the art and which refer to an amount of pharmaceutical agent effective for production of an intended pharmacological effect. Therefore, the therapeutically effective amount is an amount sufficient for reducing the manifestation of diseases to be treated. A useful assay for confirming an effective amount (e.g., a therapeutically effective amount) for a predetermined application is to measure the degree of recovery from a target disease. An amount actually administered depends on an individual to be treated. The amount is preferably optimized so as to achieve a desired effect without significant side effects. The determination of the therapeutically effective dose is within the ability of those skilled in the art.

A therapeutically effective dose of any compound can be initially estimated using either a cell culture assay or any appropriate animal model. The animal model is used to achieve a desired concentration range and an administration route. Thereafter, such information can be used to determine a dose and route useful for administration into humans.

The therapeutically effective amount refers to an amount of a decoy compound which results in amelioration of symptoms or conditions of a disease. The therapeutic effect and toxicity of such a compound may be determined by standard pharmaceutical procedures in cell cultures or experimental animals (e.g., $ED_{50}$, a dose therapeutically effective for 50% of a population; and $LD_{50}$, a dose lethal to 50% of a population). The dose ratio between therapeutic and toxic effects is a therapeutic index, and it can be expressed as the ratio of $ED_{50}/LD_{50}$. Pharmaceutical compositions which exhibit high therapeutic indices are preferable. The data obtained from cell culture assays and animal studies can be used in formulating a range of amounts for use in humans. The dosage of such compounds lies preferably within a range of circulating concentrations that include the $ED_{50}$ with little or no toxicity. Such a dosage may vary within this range depending upon the dosage form employed, the susceptibility of a patient, and the route of administration. As an example, the dose of a decoy is appropriately selected depending on the age and other conditions of a patient, the type of a disease, the type of the decoy employed, and the like. For example, the decoy is administered to brain once to several times per day in an amount of 10 to 10,000 nmole per time. The decoy is administered to a carotid artery, generally, once to several times per day in an amount of 10 to 10,000 nmole per time.

In one embodiment, the therapeutically effective amount may be typically at least 0. 01 mg/kg bodily weight,

EP 1 512 415 A1

usually at least 0.05 mg/kg bodily weight, preferably at least 0. 1 mg/kg bodily weight. In the case of knee treatment, the therapeutically effective amount may be 100 μg to 100 mg/joint, usually at least 5 mg/joint, preferably at least 10mg/ joint, more preferably at least 20mg/knee, and still more preferably at least 30 mg/joint. The therapeutically effective amount per bodily weight may be varied depending on the genus, species to be treated. In the case of a human patient, the therapeutically effective amount maybe at least 1mg/joint, usually at least 5mg/joint, preferably at least 10mg/joint, more preferably at least 20 mg/joint, still more preferably at least 30 mg/joint. In the case of administration to a knee of an osteoarthritis and rheumatoid arthritis patient, the therapeutically effective amount may be 0.01 to 500 mg/joint, usually 0.05-100 mg/joint, preferably at least 0.1-5 mg/joint. In the case of other knees, the amount of administration may be set in proportion to the volume of the cavum articulare thereof. The therapeutically effective amount can be extrapolated from monkey, rat and the like with high probability. The frequency of administration may be at any frequency, and usually can be made once per four weeks to daily. In the case of osteoarthritis and rheumatoid arthritis, the above-mentioned dosage is appropriate as a therapeutically effective amount.

In a preferred embodiment, the decoy or decoy compound of the present invention may be present in an amount of at least 0.1 mg/ml, and more preferably 1.0 mg/ml. In another preferred embodiment, the decoy or decoy compound of the present invention maybepresent in an amount of at least 2. 0 mg/ml, at least 5.0 mg/ml, at least 5.0 mg/ml, at least 20.0 mg/ml, at least 30.0 mg/ml, at least 50.0 mg/ml, at least 70.0 mg/ml, at least 100.0 mg/ml, at least 200.0 mg/ml, or more.

The exact dose and a decoy concentration in a composition are chosen by an individual physician in view of the condition of a patient to be treated. Doses and administration are adjusted to provide a sufficient level of the active portion, or to hold a desired effect. Additional factors to be considered include the severity of the condition of a disease (e.g., the size and location of a tumor; the age, weight and sex of a patient; a diet-limiting time and frequency of administration, a combination of drugs, reaction susceptibility, and resistance/response to treatment). A sustained action pharmaceutical composition may be administered every 3 to 4 days, every week, or once per two weeks, depending on the half life and clearance rate of a specific preparation. Guidance for specific doses and delivery methods are provided in publications known in the art.

Medicaments containing the thus-obtained decoy as a major component can be administered in various manners, depending on the type of disease, the type of the decoy employed, and the like. For example, the medicament can be intravascularly administered, applied to the site of a disease, administered to the disease site, or intravascularly administered to the disease site, for inflammatory diseases, or disorders or the like. For example, the medicament can be infused directly to a joint in the case of chronic articular rheumatism, osteoarthritis or the like.

In one aspect, the diseases or disorders treated by the compound of the present invention are inflammatory diseases or disorders. In another aspect, the disease or disorder treated by the present invention is an articular disease or disorder (such as arthritis).

As used herein the term "inflammation (inflammatory reaction)" refers to a basic pathological process comprising a dynamic complex of cellular and/or histological reactions caused by damage by a physical, chemical or biological acting agent, diseased blood vessels and tissues adjacent thereto. Inflammation includes processes of local reactions, morphological changes resulting therefrom, destruction and removal of injuring substances, repair and cure. Cardinal symptoms of inflammation include flare (becoming red), caumesthesia (hyperthermia), growth (swelling), ache (pain) and the like. Further, loss of function (suppression or loss of function) may arise. All these symptoms may be observed but not all the symptoms may be present. Inflammation is a local response corresponding to toxic stimulation to tissue in which micro circular systems are distributed. Toxic stimulation may be for example, foreign materials, physical energy, and the like and the damaged tissues per se may also be such inflammatory stimulation. Accordingly, inflammation is a local response in response to adverse stimulation in a tissue distributed in the microcircular system. Such adverse stimulations include, for example, foreign materials, physical energy and the like, and in addition include damaged tissue per se, which can be phlogogenic. Accordingly, inflammation in higher animals is characterized by a series of microcircular systematic responses in response to stimulation. That is, in normal inflammation, the microcircular system transiently contracts itself, thereafter expands itself, and thereby usually opens the capillary bed, which usually closes, resulting in the increased blood volume. Further, opening space of endothelial cells of veinlet regions, provokes a blood vessel penetration sthenia phenomenon, in which plasma components exudate into the interstitial tissue through the space. This blood vessel penetration sthenia takes place in two phases, and the first phase is weak response which is raised by histamine or serotonin, and the second phase which is a late-type penetration, and plays a maj or role in blood vessel penetration in inflammation. Next, polynuclear white blood cells, mononuclear cells (macrophage) , lymphoid cells, and the like, go out from the veinlet regions to the interstitial tissue. The action of an active agent produced by these plasma and cellular components facilitate the proliferation of tissue cells, thereby repair is introduced. This series of processes are described as redness, pain, fever, swelling. Basically, inflammation is a localized protective reaction, and in addition, may have a tissue damaging action. Therefore, inflammation further includes a functional disorder as a cardinal symptom. Accordingly, in detail, inflammation reactions re a complex reaction with a local tissue, involving cytopathy, circulation disorders, proliferation and the like, and therefore may refer to the global dynamic process *per se.*

As used herein, "inflammatory disease" refers to a disease which causes chronic inflammation to global diseased site including inflammatory cells such as lymphoid cells, plasma cells, acidophil, neutrophil, and the like. Such inflammatory diseases include, for example, inflammation including arthritis such as rheumatoid arthritis and osteoarthritis, nephritis, hepatitis; acute renal failure, chronic renal failure, arteriosclerosis, glomerulonephritis, pyelonephritis, urocystitis, prostatitis, urethritis, epididymitis and testitis and the like.

As used herein "articular disease or disorder" includes a disease or disorder caused in the joint, including for example, rheumatoid arthritis, osteoarthritis, periarthritis humeroscapularis, cervicobrachial syndrome and secondary arthritis and the like.

As used herein, "arthritis" refers to a disease causing inflammation in the joint. Inside the joint a cavum articulare forms from cartilage and synovial fluid, and includes bone under the cartilage, and articular capsule, bone, muscle, tendon, ligament and the like outside the synovial fluid. The joint is amenable to inflammation since the joint is always subject to mechanical stimulation when motion takes place. Causes of inflammation may be infection, trauma, allergy, dysbolism and the like. Symtoms associated thereby may be swelling of the joint, pain (arthralgia), local heat, hypomotility and the like. Pathologically, cellular infiltration into synovium, edema, proliferation of connective tissue and the like are observed.

As used herein "rheumatoid arthritis" refers to a disease characterized in chronic arthritis with unknown causes. The male-female ratio thereof is 1:4, and those of between the ages of 30 and 50 frequently contract the disease. Morbidity thereof in Japan is estimated to be 0.3-0.5 % of the total population. Pathological causes are unknown to date, however, it is suggested a number of multifactorial genetic factors particularly HLA-D4 and virus infection may be involved. Arthritis may take place in all synovial joints, and present a tendency of multiplicity and symmetry. Initially, only synovial inflammation is observed, and then advances into failure of cartilage and bone, and deformation of the cartilage, luxation occurs, and bony ankylosis occurs resulting in loss of mobility. In particular, carpusand tarsus are amenable to deformity, and ulnar malposition at articulationes metacarpophalangeae, swan-neck deformity of carpus, boutoniere deformity of carpus, and hallux valgus of tarsus are characterstically observed. Besides joints, external pericarditis, angiitis, subcutaneous tubercule, and fibroid lung and the like may be associated, and there is a bad prognosis in cases with types of angiitis such as disseminated necrotizing periarteritis, and thus such types are calledmalignant rheumatoid arthritis. Conventionally, medical treatment include the use of palliation introductory agents such as steroids, non-steoridal antiinflammatory agents, gold-agents, D-penicillamine and the like; orthopedic treatment such as synovectomy, arthroplasty, replacement arthroplasty and the like; rehabilitation treatement such as thermal treatment, exercise treatment, orthosis treatment, prosthesis treatment and the like. However, it was difficult to achieve complete cure. In particular, steroids and the like have caused problematic adverse effects.

As used herein "arthritis deformans" and "osteoarthritis" are interchangeably used to refer to a disease, wherein chronic regressive change and productive change simultaneously occur in the joints and thereby change the form of the joints. It is classified as primary osteoarthritis and second osteoarthritis. The former (primary) is found in the middle or later ages and therefor develops with mechanical stresses in addition to aging phenomena. The latter (secondary) may also be found in a younger generation, and develops subsequent to apparent causes such as joint trauma, malformation, a disease, dysbolism and the like. Pathologically, joint cartilages are gradually abraded or defected resulting in exposure of bones. At the same time, cartilage hypertrophically grows with formation of blood vessels, and spurs are formed. Growth of synovium, hypertrophy or atrophy of articular capsules, eruption of educts are observed. Conventionally, as pharmacotherapy, administration of non-steroidal antiphlogistic alangistics and intraarticular injection of steroids and the like have been conducted. On the other hand, when a lesion is advanced, as surgical treatments, arthrodesis, arthroplasty, osteotomy and prosthetic replacement arthroplasty and the like, are conducted.

Sites to be treated by the present invention may be derived from any type of organism. Organisms to be treated by the present invention include vertebrates and invertebrates, preferably mammals (e.g., primates, rodents, and the like), more preferably primates, and most preferably humans.

The composition and kit of the present invention are used typically with supervision of a physician, or without it when permitted by an authority and a law of a concerned country.

In another aspect, the present invention provides a kit for treating ischemic brain disorders. This kit comprises the decoy or the decoy compound of the present invention; and a manufacturer's instruction which provides guidelines for administration of the decoy or the decoy compound. The manufacturer's instruction describes a statement indicating an appropriate method for administrating the decoy or the decoy compound. The manufacturer's instruction is prepared in accordance with a format defined by an authority of a country in which the present invention is practiced (e.g., Health, Labor and Welfare Ministry in Japan, Food and Drug Administration (FDA) in U.S., and the like) , explicitly describing that the instruction is approved by the authority. The manufacturer's instruction is a so-called package insert, and is typically provided in a medium including, but not limited to, paper media, electronic media (e.g., web sites and electronic mails provided on the Internet).

The amount of the decoy or decoy compound of the present invention can be easily determined by those skilled in the art with reference to the purpose of use, a target disease (type, severity, and the like), the patient's age, weight, sex, and case history, the form or type of a biologically active substance in cells, the form or type of the cells, and the like.

The frequency of the method of the present invention which is applied to a subject (patient) is also determined by the those skilled in the art with respect to the purpose of use, a target disease (type, severity, and the like), the patient's age, weight, sex, and case history, the progression of the therapy, and the like. Examples of the frequency include once per day to several months (e.g., once per week to once per month). Preferably, administration is performed once per week to month with reference to the progression.

In another embodiment, the treatment method of the present invention may further comprise administrating another pharmaceutical agent. Such a pharmaceutical agent may be any medicament in the art, including any pharmaceutical agents (e.g., antibiotics and the like) known in the pharmacology field. Of course, such a pharmaceutical agent may be at least two other pharmaceutical agents. Examples of the pharmaceutical agents include those described in the latest Japanese Pharmacopoeia, the latest U. S . Pharmacopoeia, the latest Pharmacopoeias in other countries, and the like. The pharmaceutical agents may be those which preferably have an effect on cerebral ischemic diseases (e.g., antiplatelets, cranial nerve function improvers, cerebral metabolism improvers, bloodstream improver, and the like).

Molecular biological techniques, biochemical techniques, and microbiological techniques used herein are well known and commonly used in the art, and are described in, for example, Ausubel F.A. et al. ed (1988), Current Protocols in Molecular Biology, Wiley, New York, NY; Sambrook J et al. (1987) Molecular Cloning: A Laboratory Manual, 2nd Ed., Cold Spring Harbor Laboratory Press, Cold Spring Harbor, NY; Jikken-Igaku "Idenshi-Donyu & Hatsugen-Kaiseki-Jikkenho [Experimental medicine "Experimental methods for Gene introduction & Expression Analysis", Yodo-sha, special issue, 1997; and the like.

As used herein, "nucleic acid", "nucleic acid molecule", "polynucleotide", and "oligonucleotide" are herein interchangeably used to refer to a macromolecule (polymer) comprising a series of nucleotides, unless otherwise specified. A nucleotide refers to a nucleoside whose base is a phosphoric ester. The base of the nucleotide is a pyrimidine or purine base (pyrimidine nucleotide and purine nucleotide). Polynucleotides include DNA or RNA.

Further, sequences obtained by homology search through a genetic information database, such as GenBank (genome data by the human genome project) using software, such as BLAST, based on the sequence of the decoy of the present invention, also fall within the scope of the present invention.

Comparison of the identity of base sequences is herein calculated using BLAST (sequence analyzing tool) with default parameters.

As used herein, "polynucleotides hybridizing under stringent conditions" refer to conditions commonly used and well known in the art. Such a polypeptide can be obtained by conducting colony hybridization, plaque hybridization, Southern blot hybridization, or the like using a polynucleotide selected from the polynucleotides of the present invention. Specifically, a filter on which DNA derived from a colony or plaque is immobilized is used to conduct hybridization at 65°C in the presence of 0.7 to 1.0 M NaCl. Thereafter, a 0.1 to 2-fold concentration SSC (saline-sodium citrate) solution (1-fold concentration SSC solution is composed of 150 mM sodium chloride and 15 mM sodium citrate) is used to wash the filter at 65°C. Polynucleotides identified by this method are referred to as "polynucleotides hybridizing under stringent conditions". Hybridization can be conducted in accordance with a method described in, for example, Molecular Cloning 2nd ed., Current Protocols in Molecular Biology, Supplement 1-38, DNA Cloning 1: Core Techniques, A Practical Approach, Second Edition, Oxford University Press (1995), and the like. Here, sequences hybridizing under stringent conditions exclude, preferably, sequences containing only A or T.

The term "highly stringent conditions" refers to those conditions that are designed to permit hybridization of DNA strands whose sequences are highly complementary, and exclude hybridization of significantly mismatched DNAs. Hybridization stringency is principally determined by temperature, ionic strength, and the concentration of denaturing agents such as formamide. Examples of "highly stringent conditions" for hybridization and washing are 0.0015 M sodium chloride, 0.0015 M sodium citrate at 65-68°C or 0.015 M sodium chloride, 0.0015 M sodium citrate, and 50% formamide at 42°C. See Sambrook, Fritsch & Maniatis, Molecular Cloning: A Laboratory Manual (2nd ed., Cold Spring Harbor Laboratory, N.Y., 1989); Anderson et al., Nucleic Acid Hybridization: A Practical Approach Ch. 4 (IRL Press Limited) (Oxford Express). More stringent conditions (such as higher temperature, lower ionic strength, higher formamide, or other denaturing agents) may be optionally used. Other agents may be included in the hybridization and washing buffers for the purpose of reducing non-specific and/or background hybridization. Examples are 0.1% bovine serum albumin, 0.1% polyvinylpyrrolidone, 0.1% sodium pyrophosphate, 0.1% sodium dodecylsulfate ($NaDodSO_4$ or SDS), Ficoll, Denhardt' s solution, sonicated salmon sperm DNA (or another non-complementary DNA), and dextran sulfate, although other suitable agents can also be used. The concentration and types of these additives can be changed without substantially affecting the stringency of the hybridization conditions. Hybridization experiments are ordinarily carried out at pH 6.8-7.4; however, at typical ionic strength conditions, the rate of hybridization is nearly independent of pH. See Anderson et al., Nucleic Acid Hybridization: A Practical Approach Ch. 4 (IRL Press Limited, Oxford UK).

Agents affecting the stability of a DNA duplex include base composition, length, and degree of base pair mismatch. Hybridization conditions can be adjusted by those skilled in the art in order to accommodate these variables and allow DNAs of different sequence relatedness to form hybrids. The melting temperature of a perfectly matched DNA duplex can be estimated by the following equation:

$$Tm (^\circ C) = 81.5 + 16.6 (\log [Na^+]) + 0.41 (\% G+C) - 600/N - 0.72$$

$$(\% \text{ formamide})$$

where N is the length of the duplex formed, $[Na^+]$ is the molar concentration of the sodium ion in the hybridization or washing solution, % G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplex with a greater degree of base pair mismatching than could occur under "highly stringent conditions" is able to form. Examples of typical "moderately stringent conditions" are 0. 015 M sodium chloride, 0.0015M sodium citrate at 50-65°C or 0.015 M sodium chloride, 0.0015 M sodium citrate, and 20% formamide at 37-50°C. By way of example, "moderately stringent conditions" of 50°C in 0.015 M sodium ion will allow about a 21% mismatch.

It will be appreciated by those skilled in the art that there is no absolute distinction between "highly stringent conditions" and "moderately stringent conditions". For example, at 0.015 M sodium ion (no formamide), the melting temperature of perfectly matched long DNA is about 71°C. With a wash at 65°C (at the same ionic strength) , this would allow for approximately a 6% mismatch. To capture more distantly related sequences, those skilled in the art can simply lower the temperature or raise the ionic strength.

A good estimate of the melting temperature in 1 M NaCl for oligonucleotide probes up to about 20 nucleotides is given by:

$$Tm = (2^\circ C \text{ per A-T base pair}) + (4^\circ C \text{ per G-C base pair}).$$

Note that the sodium ion concentration in 6X salt sodium citrate (SSC) is 1 M. See Suggs et al., Developmental Biology Using Purified Genes 683 (Brown and Fox, eds., 1981).

A nucleic acid used as a decoy such as NF-κB may be readily isolated from a cDNA library having PCR primers and hybridization probes containing part of a nucleic acid sequence indicated by, for example, any of SEQ ID NO. 1-8. A preferable nucleic acid used in a decoy such as NF-κB, or variants or fragments thereof, or the like is hybridizable to the whole or part of a sequence as set forth in SEQ IDNO: 1-8 under low stringency conditions defined by a hybridization buffer essentially containing 1% bovine serum albumin (BSA) ; 500 mM sodiumphosphate ($NaPO_4$) ; 1mM EDTA; and 7% SDS at 42°C, and a wash buffer essentially containing 2×SSC (600 mM NaCl; 60 mM sodium citrate); and 0.1% SDS at 50°C, more preferably under low stringency conditions defined by a hybridization buffer essentially containing 1% bovine serum albumin (BSA); 500 mM sodium phosphate ($NaPO_4$); 15% formamide; 1 mM EDTA; and 7% SDS at 50°C, and a wash buffer essentially containing 1×SSC (300 mM NaCl; 30 mM sodium citrate); and 1% SDS at 50°C, and most preferably under low stringency conditions defined by a hybridization buffer essentially containing 1% bovine serum albumin (BSA); 200 mM sodium phosphate ($NaPO_4$); 15% formamide; 1 mM EDTA; and 7% SDS at 50°C, and wash buffer essentially containing 0.5×SSC (150 mM NaCl; 15 mM sodium.

"Homology" of genes refers to the degree of the identity between two or more gene sequences. Therefore, the greater the homology between two certain genes, the greater the identity or similarity between their sequences. Whether or not two genes have homology, can be studied by comparing two sequences directly or by hybridization under stringent conditions. When two gene sequences are directly compared each other, the genes have homology if representatively at least 50%, preferably at least 70%, more preferably at least 80%, 90%, 95%, 96%, 97%, 98%, or 99% of the DNA sequence of the genes are identical.

As used herein, "fragment" of a nucleic acid molecule refers to a polynucleotide having a length which is shorter than the full length of the reference nucleic acid molecule but sufficient for use at least as a factor in the present invention. Therefore, the fragment as used herein refers to a polynucleotide having a sequence length ranging from 1 to n-1 with respect to the full length of the reference polynucleotide (the length is n). The length of the fragment can be appropriately changed depending on the purpose. For example, in the case of polynucleotides, the lower limit of the length of the fragment includes 5, 6, 7, 8, 9, 10, 15, 20, 25, 30, 40, 50, 75, 100 and more nucleotides. Lengths represented by integers which are not herein specified (e.g., 11 and the like) may be appropriate as a lower limit.

"Hybridizable polynucleotide" refers to a polynucleotide which can hybridize to other polynucleotides under the above-described hybridization conditions. Specifically, the hybridizable polynucleotide includes at least a polynucleotide having a homology of at least 60% to the base sequence of SEQ ID NO: 1, preferably a polynucleotide having a homology of at least 80%, and more preferably a polynucleotide having a homology of at least 95%. Homology as described herein is represented by a score using the search program BLAST which employs an algorithm developed by Altschul et al. (J. Mol. Biol. 215, 403-410(1990)), for example. Accordingly, unless otherwise stated, comparison of similarity, identity and homology of amino acid and base sequences are calculated using FASTA, a tool for sequence analysis, with default parameter.

As used herein, "similarity" of a gene (e.g., a nucleic acid sequence, an amino acid sequence, or the like) refers to the proportion of identity between two or more sequences when conservative substitution is regarded as positive (identical) in the above-described homology. Therefore, homology and similarity differ from each other in the presence of conservative substitutions. If no conservative substitutions are present, homology and similarity have the same value.

As used herein, the "percentage of sequence identity, homology or similarity (amino acid, nucleotide, or the like)" is determined by comparing two optimally aligned sequences over a window of comparison, wherein the portion of a polynucleotide or polypeptide sequences in the comparison window may comprise additions or deletions (i.e. gaps), as compared to the reference sequences (which does not comprise additions or deletions (if the other sequence includes an addition, a gap may occur)) for optimal alignment of the two sequences. The percentage is calculated by determining the number of positions at which the identical nucleic acid bases or amino acid residues occur in both sequences to yield the number of matched positions, dividing the number of matched positions by the total number of positions in the reference sequence (i.e. the window size) and multiplying the results by 100 to yield the percentage of sequence identity. When used in a search, homology is evaluated by an appropriate technique selected from various sequence comparison algorithms and programs well known in the art. Examples of such algorithms and programs include, but are not limited to, TBLASTN, BLASTP, FASTA, TFASTA and CLUSTALW (Pearson and Lipman, 1988, Proc. Natl. Acad. Sci. USA 85(8):2444-2448, Altschul et al., 1990, J. Mol. Biol. 215(3):403-410, Thompson et al., 1994, Nucleic Acids Res. 22(2):4673-4680, Higgins et al., 1996, Methods Enzymol. 266:383-402, Altschul et al. , 1990, J. Mol. Biol. 215 (3) : 403-410, Altschul et al., 1993, Nature Genetics 3:266-272).

As used herein, the term "corresponding" gene or sequence (for example, a decoy, promoter sequences and the like) refers to a gene or sequence, which has, or is anticipated to have, a function similar to that of a predetermined gene or sequence as a reference for comparison in a species. When a plurality of such genes or sequences exist having such a function, such a corresponding gene or sequence refers to that having the same evolutionary origin. Therefore, a gene corresponding to a given gene or sequence may be an ortholog of the given gene or sequence. Therefore, genes corresponding to a mouse NF-κB gene and the like can be found in other animals (human, rat, pig, cattle, and the like). Such a corresponding gene can be identified by techniques well known in the art. Therefore, for example, a corresponding gene in a given animal can be found by searching a sequence database of the animal (e. g., human, rat) using the sequence of a reference gene (e.g., mouse NF-κB genes, and the like) as a query sequence. As such, it is intended that the present invention includes sequences corresponding to specific sequences of the present invention (for example, any one of SEQ ID NO: 1-8).

The term "Derived oligonucleotide" refers to an oligonucleotide including a derivative of a nucleotide or having a linkage between nucleotides which is not normal. Specifically, examples of such an oligonucleotide include a derived oligonucleotide in which a phosphodiester bond is converted to a phosphothioate bond, a derived oligonucleotide in which phosphodiester bond is converted to N3'-P5' phosphoramidate bond, a derived oligonucleotide in which ribose and phosphodiester bond are converted to peptide-nucleic acid bond, a derived oligonucleotide in which uracil is substituted with C-5 propynyl uracil, a derived oligonucleotide in which uracil is substituted with C-5 thiazole uracil, a derived oligonucleotide in which cytosine is substituted with C-5 propynyl cytosine, a derived oligonucleotide in which cytosine is substituted with phenoxazine-modified cytosine, a derived oligonucleotide in which ribose is substituted with 2' -O-propynyl ribose, a derived oligonucleotide in which ribose is substituted with 2'-methoxyethoxy ribose, and the like.

As used herein, "biological activity" refers to the activity which a certain factor (e.g., polynucleotide or polypeptide) has within an organism, including activity exhibiting various functions. For example, when the certain factor is a transcription factor, its biological activity includes activity to regulate transcriptional activity. When the certain factor is an enzyme, its biological activity includes enzymatic activity. As another example, when the certain factor is a ligand, its biological activity includes binding to a receptor to which the ligand corresponds. In one embodiment of the present invention, its biological activity includes activity to bind to at least one transcription factor.

As used herein, "nucleotide" refers to any naturally occurring nucleotide and non-naturally occurring nucleotide. "Derived nucleotide" refers to a nucleotide which is different from naturally occurring nucleotides but has a function similar to that of its original naturally occurring nucleotide. Such derived nucleotides are well known in the art.

As used herein, "variant" refers to a substance, such as polynucleotide, or the like, which differs partially from the original substance. Examples of such a variant include a substitution variant, an addition variant, a deletion variant, a truncated variant, an allelic variant, and the like. Allele refers to a genetic variant located at a locus identical to a corresponding gene, where the two genes are distinguished from each other. Therefore, "allelic variant" refers to a variant which has an allelic relationship with a certain gene. "Homolog" of a nucleic acid molecule refers to a nucleic acidmolecule having a nucleotide sequence having homology with the nucleotide sequence of a reference nucleic acid molecule. Representatively, "homolog" refers to a polynucleotide which hybridizes to a reference nucleic acid molecule under stringent conditions. In the case of the nucleic acid molecule of the present invention, a "homolog" is a nucleic acid molecule having a nucleic acid sequence having homology with the nucleic acid sequence of the decoy of the present invention, whose biological function is the same as or similar to the promoter of the present invention. Therefore, the concepts of "homolog" and "variant" overlap partially. Therefore, a homolog has amino acid or nucleotide homology with a certain gene in a certain species (preferably at least 60% homology, more preferably at least 80%, at least 85%, at least 90%, and at least 95% homology). A method for obtaining such a homolog is clearly understood from the description of the present specification. For example, a homolog of the decoy of the present invention may be a homologous gene in the same species or a corresponding gene in other species. Therefore, the decoy of the present invention may include all homologs of the decoy.

As used herein, the term "isolated" biological agent (e.g., nucleic acid, protein, or the like) refers to a biological agent that is substantially separated or purified from other biological agents in cells of a naturally-occurring organism (e.g., in the case of nucleic acids, agents other than nucleic acids and a nucleic acid having nucleic acid sequences other than an intended nucleic acid; and in the case of proteins, agents other than proteins and proteins having an amino acid sequence other than an intended protein). The "isolated" nucleic acids and proteins include nucleic acids and proteins purified by a standard purification method. The isolated nucleic acids and proteins also include chemically synthesized nucleic acids and proteins. Accordingly, in one embodiment, the nucleic acid of the present invention may be such an isolated nucleic acid.

In one aspect, the present invention provides a pharmaceutical composition for treating and preventing an inflammatory disease or disorder, disorders derived from such disease or disorder, as well as an articular inflammation; and a method for treating and preventing an inflammatory disease or disorder, disorders derived from such diseases or disorders, as well as an articular inflammation using the composition. In a preferable embodiment, the composition includes at least one NF-κB decoy and a pharmaceutically acceptable carrier.

In one embodiment, diseases of interest in the present invention may be at least one selected from the group consisting of rheumatoid arthritis, osteoarthritis, periarthritis humeroscapularis, cervicobrachial syndrome and secondary arthritis.

In another embodiment, the above-mentioned pharmaceutically acceptable carrier may be any carrier which is pharmaceutically acceptable, and preferably may be a liposome. More preferably, the pharmaceutical composition of the present invention is provided in a form of a HVJ-liposome.

The NF-κB decoy used in the present invention may comprise a sequence of GGATTTCCC. More preferably, the NF-κB decoy may comprise a sequence of CCTTGAAGGGATTTCCCTCC (SEQ ID NO: 1). In another embodiment, NF-κB decoy may be in a further modified form.

In a preferable embodiment, compositions and methods of the present invention may use a direct administration route to a diseased site.

Preferably, such a gene may exhibit an effect due to its expression in an inflammatory site and/or in the articulation. Examples of the gene include decoys for NF-κB, STAT-1, GATA-3, STAT-6, AP-1, E2F, Ets, and CRE. Examples of preferable decoys include 5'-CCTTGAAGGGATTTCCCTCC-3' (SEQ ID NO: 1) (NF-κB decoy), 5'-GATCTAGGGATTTCCGGGAAATGAAGCT-3' (SEQ ID NO: 2) (STAT-1 decoy), 5'-AGCTTGAGATAGAGCT-3' (SEQ ID NO: 3) (GATA-3 decoy), 5'-GATCAAGACCTTTTCCCAAGAAATCTAT-3' (SEQ ID NO: 4) (STAT-6 decoy), 5'-AGCTTGTGAGTCAGAAGCT-3' (SEQ ID NO: 5) (AP-1 decoy), and 5'-AATTCACCGGAAGTATTCGA-3' (SEQ ID N0: 6) (Ets decoy), 5'-TGACGTCA-3' (CRE decoy), or oligonucleotides containing complements thereof, mutants thereof, or compounds containing them therein. In a preferred embodiment, the pharmaceutically acceptable carrier may be a liposome.

In the present invention, it is demonstrated that *in vivo* transfection with a cis-element decoy binding to a transcription factor NF-κB is used for ameliorating inflammation such as rheumatoid arthritis.

In a conventional treatment of inflammation, steroids or non-steroidal anti-inflammatory agents are often used. However, use of steroids results in atrophy of the adrenal cortex, and causes withdrawal syndrome, renal failure, diabetes, moon face as a global adverse effect, and steroidal contusion, steoridal suffusion, skin dermatrophia, polytrichia, infectious diseases and the like as a local adverse effect.

Further, target genes for NF-κB include apoptosis-related genes (including TP53 (see Wu H, Lozano G., J Biol Chem. 1994, 269:20067-74) and c-myc (LaRosa FA, Pierce JW, Sonenshein GE., Mol Cell Biol. 1994; 14: 1039-44)). Therefore, a gene therapy according to the present invention using decoy against the NF-κB binding site suppresses gene expression relating to inflammatory responses and the articulation damage (including apoptosis).

Decoy treatment has a number of benefits, including instant effect, low cost, and less or no complex diseases. Gene therapy using a naked E2F decoy has already been tried in a clinical situation for preventing vein-graft diseases (see Mann MJ. Whittemore AD, Donaldson MC, Belkin M, Conte MS, Polak JF et al., Lancet.1999:354:1493-8).

As described above, it should be noted that the application of decoys in the field of inflammatory diseases and arthritis disorders are shown and the use thereof, in the following description.

Hereinafter, the present invention will be described by way of examples, and the following examples are provided only for illustrative purposes. Therefore, the scope of the present invention is limited only by the claims, but not the examples.

EXAMPLES

In the following Examples, exemplary typical materials and methods for use are presented.

(Example 1: Effects of NF-κB decoys on collagen arthritis models using apes)

This example tested effects of NF-κB decoy in a collagen arthritis model using apes. Actions against a type II collagen arthritis model in apes, by administering NF-κB to the intraarticular site were tested. Further, effectiveness and toxicity thereof were compared with intra-articular administration of steroids. No GLP was applied to the present test.

Test substances used are as follows:

1) Test substances: NF-κB decoyoligonucleotides (hereinafter also described as NF-κB decoy) (AnGes MG), (100 mg/vial, kept frozen at -20°C) . The sequence of ODN used is NF-κB decoy ODN, 5'-CCTTGAAGGGATTTCCCTCC-3' (SEQ ID NO: 1).

2) Positive controls: Betametasone acetate, sodium betametasone phosphate (Rinderon suspension, hereinafter described as Rinderon) (SHIONOGI) (composition: betametasone acetate 2mg, sodium betametasone phosphate 0.66 mg/0.5mL/ampoule)

3) sensitization substance: bovine type II collagen (Collagen Gijutsu Kenshukai = Collagen Technology Instutute) (-20°C, kept dark)

4) Adjuvants: Freund Complete Adjuvant (Becton Dickinson and Company) (kept dark)

5) Medium-1: saline (Otsuka Seiyaku Kojo) (kept at room temperature)

6) Medium-2: 5 mmol/L acetate saline solution (manufactured by Wako Pure Chemical = acetate, Otsuka Seiyaku Kojo =saline) (kept refrigerated).

(Preparation and administration of test substances and positive control substances)

Preparation and administration of test substances and positive control substances are as follows:

NF-κB decoy: it was solved into a vial of test substance by adding saline to dilute into 6, 20 and 60 mg/mL for use and discard remaining solution.

Rinderon: Positive control substances in ampoules were used diluted with saline. Betametasone acetate was prepared to be 0.2 mg/mL. One ampoule (0. 5mL) was diluted to 10mL (twenty times dilution) and remaining solution was discarded.

As a test animal, forty apes (macaque; purpose-bred) , body weight: 2-3.5 kg (at the start of conditioning) , ages 2-3 years, origin: China (place of breeding: Guangxi Primate Center of China 14 Yongwu Road, Nanning, Guanxi 530001, China), importer: China National Scientific Instruments & Materials Import/ Export Corporation) were conditioned and thirty among the same were subjected to administration treatment. Generally, apes are considered to be a test animal similar to a human. Articular forms thereof are similar to those of a human, and size thereof is similar, as well as evaluation being easy. Therefore, apes are used as a model. With respect ethics of animal use, the present examples were conducted in accordance with the provisions regarding the ethics of animal experiments of Shin-Nihon kagaku.

Breeding was conducted as follows: breeding temperature was set at 26±2 °C, breeding moisture was set to 50 ±10 %. Ventilation rounds were set to 15 times/hour, and illumination was set to use artificial illumination of twelve hours a day (6:00 a.m. to 6:00 p.m.). Breeding cages used are made of stainless steel and are compatible to the USDA provisions and the number to be housed is 1 ape/cage. Feed used are solid feed (Teklad Global Certified 25% Protein Primate Diet, Harlan Sprague Dawley Inc.), and about 108 g (about 12 g x 9) were given once a day at 3:00 p.m. and the following day at 9:00 a.m., remaining feed was collected. One day before hematological test, blood biochemical test, administration and autopsy, remaining feed were collected at about 5:00 p.m.

Drinking water was given freely using automatic feed water supply (Okasaki Sangyo Kabushiki Kaisha) with water compatible to water quality standard as defined under Water Works Law.

Cleaning was conducted daily with water inside the room and sewage vessels in cages were washed with water.

Identification of animals was as follows: identification of individuals: tattoo with animal number on the chest (Spaulding Special Electric Tattoo Marker, Spaulding & Rogers Mfg., Inc.) was made. For identification of cages, color cage cards (with test number, group number, amount of administration, sex and animal number are described) were used.

Conditioning:

Forty quarantined female apes were selected for weighing. Conditioning periods were set to three weeks before the start of administration, and therebetween general condition were observed once a day, and measurement of food intake was conducted once a day for seven days before the start of administration, and weighing was conducted at the grouping date and the date of end of conditioning once for each. Hematological and blood biochemical tests were conduced once. The detailed methods are referred to "17. OBSERVATION AND TEST ITEMS".

Animal grouping:

Regarding animals in which no abnormality was observed from the start of conditions and until the previous date of the start of the sensitization, grouping was conducted so that each group has approximately the same average body weight one day before the start of the sensitization (each group has five males; 30 in total) . Animals not used for the test were excluded from the test as remaining animals and were maintained as reserve breeding animals.

Apes were anesthetized with ketamine (ketamine hydrochloride. about 10 mg/kg, Sigma Chemical Co., intramuscular injection, optionally additional administration was provided), and then administration was made at ankle joint, knee joint and intracarpus joint (right and left for each, six sites in total). Amount of administration was 0.5 mL/joint (ankle joint and intracarpus joint) and 1.0mg/joint (knee joint). The rounds of administration were as follows: Groups 1-3 and 6: six times (once in two weeks); Group 4: five times (once in two weeks; fifth administration was conducted four weeks after the fourth round (on day 70); Group 5: three times (once in four weeks), (third administration was conducted about six weeks after the second administration; on day 72). The rounds of administration of apes are selected for prognosis of a clinical application in human. Administration time was 10.00 a.m. to noon.

Test methods:

1) Preparation of collagen articulitis model

Thirty female macaques were used. Bovine type II collagen 24 mg/vial was added to 5mmol/L acetate saline 6 mL, agitated for 24 hours in a refrigerator (4 °C), and solubilized. After mixing equal amounts of the solution and Freund's Complete Adjuvant, emulsion was made by full suspension. A disposable syringe and needle was used for administration of 2mL of emulsion (type II collagen, 4mg) per ape intradermically into the back to sensitize (primary sensitization). One week after the primary sensitization, the emulsion of the same composition was administered intradermally into the back for boost immunization. Administration of emulsion was conducted under ketamine e anesthesia (ketamine e hydrochloride about 10mg/kg, Sigma Chemical Co., intramuscular injection, optionally additional administration, amount of administration was recorded).

Test group configuration

Control group: 1; test substances: 4 groups; and positive control substance: 1 group (total six groups) were used for the following experiments. Regimen is shown in Figure **3**.

TABLE 1

| Group | Test substance | Administration dose* per joint (ankle and carpus) | Administration volume* per joint (ankle and carpus) | Total administration mount per ape | Animal number |
|---|---|---|---|---|---|
| 1 | Control ( Saline ) **(two-week interval) | 0 | 0.5 RILL | 0 | 1-5 |
| 2 | NFκB decoy 3 mg (two-week interval) | 3 mg | 0.5 mL | 144 mg | 6-10 |
| 3 | NFκB decoy 10 mg(2 two-week intervals) | 10 mg | 0.5 mL | 480 mg | 11-15 |
| 4 | NFκB decoy 30 mg (two-week intervals)*** | 30 mg | 0.5 mL | 1440 mg | 16-20 |
| 5 | NFκB decoy 30 mg (four-week intervals)*** | 30 mg | 0.5 mL | 720 mg | 21-25 |
| 6 | Steroid (Rinderon) administration group (two-week intervals) | 0.1 mg | 0.5 mL | 4.8 mg | 26-30 |

* double amount was given for knee joint

** administration of saline was conducted in a similar manner as in the test substances

*** fifth administration for Group 4 was conducted four weeks after the fourth round (on day 70), third administration for Group 5 was conducted about six weeks after the second administration (on day 72).

In a test of a type II collagen articulitis model in apes, effective ratio was 3/4 in administration at two weeks intervals of NF-κB decoy 10mg/joint. Scrambled decoys were observed to be effective ratio of 1/4. Accordingly, in consideration of safety in a clinical situation, the maximum amount was set to 30mg/joint and subsequent amounts were set to 10 and 3 mg/joint with the common ratio of 3. Further, since NF-κB decoy shows a long period of effectiveness when it was incorporated into the cellular nucleus, a group where 30mg was administrated once four weeks, was also set. As the dose of a steroid (Rinderon) is 2mg/joint, therefore, the dose for apes was set to 1/20 thereof in view of bodily weight.

Observation and test parameters were as follows. The starting date of administration was set to Day 0 of administration, and the week when the administration started was set to Week 1 of administration.

1) General conditions: Observation was made more than once a day during the period of administration (in the morning and in the afternoon), and once a day during autopsy was conducted for all the cases. Swelling of joints were evaluated with four stages (no abnormality, light swelling, moderate swelling and high swelling), and the results thereof were recorded.

2) Feed amounts: Seven days before the administration started, the number of feed and remaining thereof were recorded and the amount of feed (g) was calculated for all the cases, and weekly average therefor was calculated as the feed amount per day for the week.

3) Bodilyweight: Bodily weights were measured for all cases with electronic balance (HP-40K, A and D, Inc.) one day before sensitization (grouping day), one day before administration (final conditioning day) , once a week after administration start, and date of autopsy.

4) Hematological tests:

Test frequency:

Groups 1-3 and 6: Once during conditioning period, Days 0, 14, 28, 42, 56 and 70 (before administration) anddateofautopsy.
Groups 4 and 5: Once during conditioning period, Days 0, 14, 28, 42, 56, 70 (before administration) and 84.

Number of examples: All cases in each group

Method of blood sampling: Blood sampling was conducted using syringe from femoral vein. Whole blood treated with EDTA-2K for anticoagulation, was used.

Test items: as in the following table:

TABLE 2

| Test parameter | Method for measurement | Apparatus |
|---|---|---|
| Red cell number | Two angle laser flow cytometry method | ADVIA120 * |
| White cell number | Two angle laser flow cytometry method | ADVIA120* |
| Hematocrit | Calculated by the known formula | ADVIA120* |
| Hemoglobin concentration | Cyanmethemoglobin method | ADVIA120* |
| Platelet number | Two angle laser flow cytometry method | ADVIA120* |
| MCV | Two angle laser flow cytometry method | ADVIA120* |
| MCH | Calculated by the known formula | ADVIA120* |
| MCHC | Calculated by the known formula | ADVIA120* |
| Reticulocyte number | Flow cytometry method using RNA staining | ADVIA120* |
| White blood cell classification | Peroxidase staining method and two angle laser flow cytometry method | ADVIA120* |

*:Total hematological test apparatus(ADVIA120,BAYER DIAGNOSTICS MANUFACTURING LTD)

5) Blood biochemical tests:

Test frequency:

Groups 1-3 and 6: Once during conditioning period, Days 0, 14, 28, 42, 56 and70 (before administration) and date of autopsy.
Groups 4 and 5: Once during conditioning period, Days 0, 14, 28, 42, 56, 70 (before administration) and 84.

Number of examples: All cases in each group

Method of blood sampling: About 1.2 mL of blood was bled using syringe from femoral vein. After allowing the sample to stand still for 20-40 minutes at room temperature, centrifugation was conducted at 3000 r.p.m. for 15 minutes and the resultant serum was used.

Test items: CRP (turbidity immunological assay (TIA) ; see Clin Chim Acta 1977 May 2; 76(3): 377-88) was measured.

6) X-ray images:

Radiographical analysis were conducted using a portable X-ray machine (DOP-82S-120-D type, Hitachi Medico, Inc.) with respect to carpus joints, knee joints, and tarsus joints (right and left for each) under ketamine anethetics (using ketamine hydrochloride about 10 mg/kg, Sigma Chemical Co., intramuscular injection, optionally additional injection was given, amount of administration was recorded), for all the cases in Groups 1-3 and 6 (before primary sensitization) on Days 0, 14, 28, 42, 56 and 70 (before administration), and one day before or the date of autopsy, and for all the cases in Groups 4-5 on Days 0, 14, 28, 42 and 70 (before administration), and the one day before or date of autopsy. Radiation was given to the carpus joints from the front (simultaneously for right and left), to the knee joints from the front (simultaneously for right and left) and from the sides (laterally for each, radiated from inside, intorted 60°.), and to the tarsus joints from the sides (laterally for each, radiated from inside) to obtain photographs. Radiation distances were set to substantially the same. Marker was used for X-ray photographs.

7) Bone volume measurement:

For all cases, bone volumes of the distal member of the femur and the proximal member of the tibia (right and left for both) using double energy X-ray absorption analyzer (DPX-alpha, LUNAR) before starting administration and at the time of dissection.

8) Measurement of blood anti-collagen antibody value:

Blood sampling frequency:

| | |
|---|---|
| Groups 1-3 and 6: | Before primary sensitization, Days 0, 14, 28, 42, 56 and 70 (before administration) and date of autopsy |
| Groups 4-5: | Before primary sensitization, Days 0, 14, 28, 42 and 70 (before administration) and Day 84. |
| Number of Examples: | All the cases of each group |
| Volume of bleed: | About 1mL (about 0.4 mL as serum). About 7. 5 mL (about 3 mL as serum) were further taken on the date of autopsy and Day 84 of the administration |
| Methods of blood sampling: | Blood sampling is made from femoral vein using a syringe. Blood is left to stand for twenty to forty minutes at room temperature, and centrifuged at 3000 r.p. m. for fifteen minutes to obtain serum. Resultant serum was kept frozen at -20 °C until being sent (the additionally taken serum (about 3 mL) on the date of autopsy and Day 84 of administration was kept -80 °C). The serum was sent to the test requester (AnGes MG, Inc.) in the presence of dry ice. |

Method of Measurement: Bovine type II collagen is coated onto a microplate to form solid phase. Serum obtained for measuring antibody value was used as a primary antibody, and anti-IgG (Fc) peroxidase labeled goat antibody, and simian IgM (Fc) peroxidase labeled goat antibody were used as a secondary antibody for conducting ELISA measurement. A plate reader (SEM 3400, Iwaki Glass, Inc.) was used for measurement after developing color.

9) Intraarticular endoscopy

Test frequency:     Weeks 4, 8 and 12 of administration

Number of Examples:

Week 4 of administration: One for each of no treatment group, control group, NF-κB 30 mg group (two-week intervals), and steroid administered group (Animal Numbers 4, 19 and 29)

Weeks 8 and 12 of Adminsitration: One for no treatment group, two for control groups (Animal Numbers 2 and 4), one for NF-κB 10 mg group (two-week intervals), one for NF-κB 30 mg group (two-week intervals), and one for steroid administered group (Animal Numbers 13, 19 and 29)

Test methods: Intraarticular endoscope was inserted into the knee joint under inhalation or medetomidine hydrochloride anesthesia.

10) autopsy:

Dead examples were autopsied soon after weighing, and carpus joints, knee joints and ankle joints (right and left) were X-ray photographed. Further, organs to be stored for joint observation and histopathological examination were stored in 10 % neutral buffered formalin. For the remaining organs and tissues, gross observation was conducted. Tissues and Organs in which changes were observed by the gross observation were stored in formalin depending on the judgment of the person in charge of autopsy. Living examples were received 0.4 mL/kg of pentobarbital aqueous solution (64.8 mg/mL) (Tokyo Kasei Kogyo Co. Ltd.) on the final date of observation by administering to the forearm cephalic vein. After euthanasia by exsanguination under anesthesia, the animal was fixedby perfusing 10 % neutral buffered formalin, and observed in the same manner as for the dead examples. When abnormalities were observed, photographs were taken for exemplary animals in which changes were considered to be due to the administration of the test substance.

11) Histopathological examination:

Number of Examples:     All the examples

Specimen preparation: Obtaining organs after fixation by perfusion (refer to the organs to be stored). Joints (joint synovium and joint cartilage) of carpus, knee and tarsus (right and left for each) were incised, and decalcification, embedding in paraffin, and thin sectioning have been conducted by Shin Nippon Biochemical Laboratories, Ltd. One for each specimen for H.E. staining and two for non-stained specimen were sent to the examiner. When necessary to inspect organs and tissues in which changes were observed by gross observation, specimen for H.E. staining were prepared in accordance with well known and routine technology in the art.

Organs to be stored: Heart, Lung*, Bronchus, Liver, Cholecyst, Kidney*, Adrenal Gland*, Carpus Joint*, Knee Joint*, Tarsus Joint*, and Abnormal sites * refers to the organs both lateral of which were obtained for storage. Storage of Specimen: The above-mentioned organs and (a portion for large organs) were stored after vacuuming.

(Results)

The result of the present Example is shown in Figure **1**. In Figure **1**, changes of the joints by the decoys of the present invention in simian collagen arthritis models are shown by X-ray photographs. No effects were observed by no treatment and scrambled decoys, whereas, the models received the treatment of the decoys of the present invention were observed to have significant improvement such as suppression of synovial inflammation change, joint welling, joint cartilage and osteoclasis of joints.

(Example 2: Effects of NF-κB on osteoarthritis model using rats)

This example addresses the effects of NF-κB decoys on osteoarthritismodle using rats in combination of hyarulonic acid. A combination drug of NF-κB and hyaluronic acid was intraarticularly administered to a rat to address the action thereof on a rat osteoarthritis model.

After incision of a crucial ligament of the rat, NF-κB decoy labeled with FITC was intraarticularly administered to investigate transfer characteristics into the cartilage one and two months later. The present tests were conducted as non-GLP tests.

The test substances used were as follows:

1) Test substance 1: NF-κB decoy oligonucleotide (hereinafter described as NF-κB decoy) (AnGes MG), (100 mg/ vial, kept frozen (-20 °C))

2) Test substance 2: Low molecular hyaluronic acid (ARTS (trademark); SEIKAGAKU CORPORATION; stored frozen at -20°C)

3) Test substance 3: HVJ-E introduced NF-κB (AnGes MG) (0. 1 mg (NF-κB), 2000 HAU (HVJ-E)), (stored frozen at -20°C). HVJ virus-liposome complex were prepared as described in the reference(Morishita R, Sugimoto T, Aoki M, Kida I, Tomita N,Moriguchi A et al., Nat Med.1997;3:894-9). In brief, phosphatidyl serine (PS), phosphatidyl choline (PC) and cholesterol (Chol) were mixed at the ratio by weight of 1: 4.8:2. The lipid mixture (10 mg) was deposited on the sides of a flask by removal of a solvent (tetrahydrofuran) in a rotary evaporator. Dried lipid was hydrated in 200 μl of physiological saline containing 200 μg of ODN. Liposomes were prepared by shaking and sonication. Liposome suspension (0.5 mL, containing 10 mg of lipids) was mixed with HVJ (10,000 hemagglutinating units) inactivated in physiological saline having a total volume of 4 mL. The mixture was incubated at 4°C for 5 minutes and then for 30 minutes with gentle shaking at 30°C. Free HVJ was removed from the HVJ-liposomes by density gradient centrifugation. The top layer of the sucrose gradient was collected for use. The sequences of ODN are the following: NF-κB decoy ODN, 5' -CCTTGAAGGGATTTCCCTCC-3' (SEQ ID NO: 1), and scrable decoy ODN 5' -CATGTCGTCACTGCGCTCAT-3' (SEQ ID NO: 7); and 5'-ATGAGCGCAGTGACGACATG-3' (SEQ ID NO: 8).

(Preparation and Administration of Test Substances)

(Preparation Methods)

As for test substance 1, twentymg of NF-κB decoy were weighed, dissolved at room temperature in saline and diluted if necessary for use. Remaining solution was discarded.

As for test substance 2, decoy was prepared in a solution comprising 25 mg of sodium hyaluronic acid in one ampoule (2.5 ml).

As for scramble decoy, it was prepared as for NF-κB decoy.

The above-mentioned preparation was conducted before use.

As for test animals, fifty male rats (Fischer 334 lines) (body weight: 250-300 g at the start of test, 12 week old, Japan Charles River Inc., Hino, Gamo-gun, Shiga) were conditioned and 43 animals thereof were subjected to administration. Generally, rats are widely used as experimental animals, and operation of knee joints is readily conducted and evaluations of pharmacological effects are easy, and therefore were used as osteoarthritis model. As for ethics of animal use, the inventors conducted experiments according to the provisions for ethics of animal experiments defined by Osaka University.

Animal breeding was conducted as follows. Breeding temperature was set to 26±2 °C, breeding humidity was set to 50±10 %. Ventilation frequency was set to 15 times/hour, and artificial illumination was given for 12 hours per day (from 6:00 a.m. to 6:00 p.m illumination) . Breeding cage used was made of stainless steel, and the sizes thereof were in accordance of USDA standard. Number of animals to be housed was set to five/cage. Feed used were solid feed which was freely given to the animals.

Drinking water was water in accordance with the water quality standard defined under Water Works Law prepared by automatic water feeding apparatus (Okazaki Sangyo Co. Ltd.)

Identification of animals:

Identification of individuals: Pigment application
Identification of cages: group, amount of administration, were used cage cards (test number, animal number are described)

Medical inspection and conditioning:

Forty-three male rats were selected from those which received medical inspection, and were weighed. One week before operation was determined to be a conditioning period in which general condition was observed. Animal grouping: animals (forty-three male animals) which did not show any abnormalities during the conditioning period were randomly selected on the final date of the conditioning. No animal grouping was conducted. Animals not used for examination has been euthanized as excessive animals.

Preparation of osteoarthritis models:
Animals (thirty-three) which did not show abnormalities during the conditioning period were randomly selected. The animals received intramuscularly 300 μl of an aqueous solution of Ketalar (ketamine) and Celactal (xylazine) 2:1. Under such anesthesia, knee joint was exposed and the crucial ligament was dissected. Animals not used for examination were euthanized as excessive animals.

Administration was conducted as follows. Intraarticular administration was selected as an administration route. This is in order to conduct in accordance with the clinical administration route. Direct injection into a rat knee joint under anesthesia was selected as a method for administration. Intraarticular administration is conventionally used for rat treatments. Administration amount is 0.05 mL/joint, and the frequency of administration was set to 8 times per week. The frequency was selected for prediction of a human clinical situation.

TEST METHODS:

Dosage and number of animals: animals were divided into three groups, and models were created. Operation number per round was averaged. Test group constitution and animal numbers are shown below. Administration schedule is presented in Figure 4.

TABLE 3

| FIRST OPERATION GROUP | | | | |
|---|---|---|---|---|
| Administration groups | Administration amount (as NFκB decoy) per joint | Administration dose per joint | Total administration dose per animal | Animal number |
| Control (Saline) | 0 | 0.05ml | 0 | 1~ 2 |
| NFκB decoy 10mg/ ml | 0.5mg | 0.05ml | 8mg | 6-10 |

TABLE 3 (continued)

| FIRST OPERATION GROUP | | | | |
|---|---|---|---|---|
| Administration groups | Administration amount (as NFκB decoy) per joint | Administration dose per joint | Total administration dose per animal | Animal number |
| NFκB decoy 10mg/ml +HA | 0.5mg | 0.05ml | 8mg | 11-15 |

TABLE 4

| Second Operation Group | | | | |
|---|---|---|---|---|
| Administration groups | Administration amount (as NFκB decoy) per joint | administration dose per joint | total administration dose per animal | Animal number |
| Control (Saline) | 0 | 0.05ml | 0 | 3 ~4 |
| Scramble Decoy 10mg/ml+HA | 0mg | 0.05ml | 0mg | 16 ~ 20 |
| NFκB decoy 10mg/ml +HVJ-E | 0.1mg | 0.05ml | 1.6mg | 21 ~ 25 |

TABLE 5

| THIRD OPERATION GROUP | | | | |
|---|---|---|---|---|
| Administration groups | Administration amount (as NFκB decoy) per joint | Administration dose per joint | Total administration dose per animal | Animal number |
| Control (Saline) | 0 | 0.05ml | 0 | 5,43 |
| NFkB decoy 10mg/ml +HVJ-E+HA | 0.1mg | 0.05ml | 1.6mg | 26 ~ 30 |
| HA | 0mg | 0.05ml | 0mg | 31 ~ 35 |
| FITC-NFκB | 0.5mg | 0.05ml | 1.0 | 36 ~ 42 |

In tests of type II collagen arthitis ape models, NF-κB decoy 10mg/joint was administered at two-week intervals resulted in the effectiveness of 3/4, whereas it was observed that the use of scrambled decoy resulted in effectiveness of 1/4. Since the maximum dose that can be intraarticulary administered into a rat is 0.05 mL, the dose to be administered to rat joint corresponded to 10mg/ml of the simian effective dose, was set to 0.5 mg/joint. Further, scrambled decoy, the DNA sequence of which was randomized against NF-κB decoy, was administered at the same amount.

Hyaluronic acid used in clinical situations was administered in combination with NF-κB decoy, the effectiveness of the NF-κB decoy was compared. HVJ-E used was prepared by inactivating HVJ-virus with Triton and removing RNA thereof and then including NF-κB decoy into the virus after mixing with NF-κB decoy 10mg/mL.

The dose of including NF-κB decoy into an HVJ-E is 25600HAU (1HAU=1x10$^6$ viruses)/mg of NF-κB decoy, about 2000 HAU were administered per joint.

In such a case, the amount of administration is calculated to be 0.1 mg. Therefore HVJ-E included NF-κB decoy was determined.

Observation and examination items (date of administration was set to Day 0) were as follows:

1) General conditions: Observation was made one day before administration, just after the administration on the date of administration, daily thereafter and once on the date of autopsy, together with the survival or death regarding all cases. On the date of administration, observation was optionally conducted in a frequent basis:

2) Body weight: Weighed on the dates of operation, administration, and thereafter once per week, and on the date of autopsy for all cases using an electronic balance (EP-41KA or HP-40K available from A and D Co. Ltd).

3) Hematological test:

Number of examples: all case
Date of blood sampling: at the time of autopsy
Method of blood sampling: Blood sampling was conducted using a syringe. Whole blood treated with EDTA-2K for anticoagulation was used.

The test items were as follows:

TABLE 6

| Test items | Measuring methods | apparatus |
|---|---|---|
| Erythrocyte number | Resistance detection | E-4000 * |
| White blood cell number | Resistance detection | E-4000 * |
| Hematocrit value | Pulse detection | E-4000 * |
| Hemoglobin concentration | Sodium lauryl sulfate, hemoglobin method | E-4000 * |
| Platelet number | Resistance detection | E-4000 * |
| MCV | Calculated | E-4000 * |
| MCH | Calculated | E-4000 * |
| MCHC | Calculated | E-4000* |
| Reticulocyte count | Brecher method | HEG-120A ** |
| Differential white blood count | Wright staining method | HEG-120A ** |

*: multi-item automatic blood cell counting apparatus (E-4000, Sysmex Co. Ltd.)

**: Blood cell automatic analyzing apparatus (MICROX HEG-120A, OMRON)

4) Blood Biochemical Test:

Case Number: All cases
Date of blood sampling: at autopsy
Blood sampling method: After blood sampling, left to stand for 40-60 minutes at room temperature, and subjected to centrifugation at 3000 rpm for 15 minutes to obtain serum for use.
Test items: CRP measured by immunological turbidimetry.

5) X-ray images:

Small animal X-ray systems (portable X-ray apparatus, DOP-82S-120D, Hitachi Medico) were used for X-ray photography of posterior limb under anesthesia at autopsy.

6) Autopsy: Dead examples were autopsied soon after weighing, and carpus joints, knee joints and ankle joints (right and left) were X-ray photographed. Further, organs to be stored for joint observation and histopathological examination were stored in 10 % neutral buffered formalin. For the remaining organs and tissues, gross observation was conducted. Tissues and Organs in which changes were observed by the gross observation were stored in formalin depending on the judgment of the person in charge of autopsy. Living examples were received 0.4 mL/kg of pentobarbital aqueous solution (64.8 mg/mL) (Tokyo Kasei Kogyo Co. Ltd.) on the final date of observation by administering to the forearm cephalic vein. After euthanasia by exsanguination under anesthesia, the animal was observed in the same manner as for the dead examples.

7) Histopathological examination:

Sample Number: Knee joints of a posterior limb were taken from all the examples and after fixation, embedded in paraffin, and thin sections were prepared according to routine methods in the art and examination was conducted.

Preparation and examination method for specimen:

Knee joints of posterior limbs were fixed with 10 % neutral buffered formalin. After sectioning specimens after fixation, the specimens were embedded in paraffin and thin sections were prepared and H.E. staining was conducted and staining observed by microscopy. If specific staining was necessary, the person in charge of the examination judged for conduction of such.

Organs to be stored: Knee joints
Photographs: Photographs were taken of representatives of knee joints.

(Results)

In Figure **2**, effects of NF-$\kappa$B decoys on osteoarthritis rat model are shown with section slide photographs of joints. Healthy donors, collagen derived arthritis models, and the effects of NF-$\kappa$B decoys (10 mg, 30 mg administrations) are shown. The administration of 10 mg of decoys has already significant improvement, and the administration of 30 mg of decoys eliminated the joint inflammation. This effect has exceeded that of steroids. Further, steroid treatments are associated with adverse effects specific to steroids, however, when using the decoy of the present invention, no such significant adverse effects were found.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

INDUSTRIAL APPLICABILITY

A pharmaceutical composition for treating or preventing an inflammatory disease or disorder using a decoy is provided. Further, manufacture of compositions, virus constructs, kits, and medicaments used in the treatment method of the present invention are fully industrially applicable. The present invention is considered to have industrial applicability or utility since for example pharmaceutical industries can produce the composition of the present invention as their business other than medical doctors. The method of the present invention is useful for clinical trials, which are a purpose of a business, in addition to pure method for medicine, and thus it is industrially applicable. Further, indirect or direct working of the method for treatment of the present invention is considered to be fully applicable in the vicinity of the medicine, and thus it is industrially applicable or useful.

**Claims**

1.  A pharmaceutical composition for treating and preventing an inflammatory disease or disorder, comprising:

    at least one NF-κB decoy, and
    a pharmaceutically acceptable carrier.

2.  The composition according to Claim 1, wherein said disease or disorder is at least one selected from the group consisting of nephritis, hepatitis, arthritis, acute renal failure, chronic renal failure, arteriosclerosis, glomerulonephritis, pyelonephritis, urocystitis, prostatitis, urethritis, epididymitis and testitis.

3.  The composition according to Claim 1, wherein the pharmaceutically acceptable carrier is a liposome.

4. The composition according to Claim 1, wherein the NF-κB decoy comprises a sequence of GGATTTCCC.

5. The composition according to Claim 1, wherein the disease or disorder is osteoarthritis or rheumatoid arthritis.

6. The composition according to Claim 1, which is adapted for administration to a knee joint.

7. The composition according to Claim 6, wherein the NF-κB decoy comprises at least 10 mg.

8. The composition according to Claim 6, wherein the NF-κB decoy comprises at least 30 mg.

9. A pharmaceutical composition for treating and preventing an articular disease or disorder, comprising:

   at least one NF-κB decoy, and
   a pharmaceutically acceptable carrier.

10. A composition according to Claim 9, wherein said disease or disorder is at least one selected from the group consisting of rheumatoid arthritis, osteoarthritis, periarthritis humeroscapularis, cervicobrachial syndrome and secondary arthritis.

11. A composition according to Claim 9, wherein said pharmaceutically acceptable carrier is a liposome.

12. A composition according to Claim 9, wherein said NF-κB decoy comprises a sequence of GGATTTCCC.

13. A composition according to Claim 9, wherein said disease or disorder is osteoarthritis or rheumatoid arthritis.

14. A composition according to Claim 9, wherein said disease or disorder is rheumatoid arthritis, and said decoy comprises at least a therapeutically effective amount.

15. A composition according to Claim 14, wherein said therapeutically effective amount is at least 0.01 mg/kg bodily weight.

16. A composition according to Claim 9, which is adapted to administration into a knee joint.

17. A composition according to Claim 16, wherein said NF-κB decoy comprises at least 10mg.

18. A composition according to Claim 16, wherein said NF-κB decoy comprises at least 30mg.

19. A method for treating or preventing inflammatory disease or disorder, comprising:

   administering to a patient a composition comprising:

      at least one NF-κB decoy; and
      a pharmaceutically acceptable carrier.

20. A method according to Claim 19, wherein said disease or disorder is at least one selected from the group consisting of nephritis, hepatitis, arthritis, acute renal failure, chronic renal failure, arteriosclerosis, glomerulonephritis, pyelonephritis, urocystitis, prostatitis, urethritis, epididymitis and testitis.

21. A method according to Claim 19, wherein the pharmaceutically acceptable carrier is a liposome.

22. A method according to Claim 19, wherein the NF-κB decoy comprises a sequence of GGATTTCCC.

23. A method according to Claim 19, wherein the disease or disorder is osteoarthritis or rheumatoid arthritis.

24. A method according to Claim 19, wherein said composition is adapted for administration to a knee joint.

25. A method according to Claim 24, wherein said composition comprises at least 10 mg of the NF-κB decoy.

26. A method according to Claim 24, wherein said composition comprises at least 30 mg of the NF-κB decoy.

27. A method for treating and preventing articular disease or disorder, comprising:

administering to a patient a composition comprising:

at least one NF-κB decoy; and
a pharmaceutically acceptable carrier.

28. A method according to Claim 27, wherein said disease or disorder is at least one selected from the group consisting of rheumatoid arthritis, osteoarthritis, periarthritis humeroscapularis, cervicobrachial syndrome and secondary arthritis.

29. A method according to Claim 27, wherein said pharmaceutically acceptable carrier is a liposome.

30. A method according to Claim 27, wherein said NF-κB decoy comprises a sequence of GGATTTCCC.

31. A method according to Claim 27, wherein said disease or disorder is osteoarthritis or rheumatoid arthritis.

32. A method according to Claim 27, wherein said disease or disorder is rheumatoid arthritis, and said decoy comprises at least a therapeutically effective amount.

33. A method according to Claim 27, wherein said therapeutically effective amount is at least 0.01 mg/kg bodily weight.

34. A method according to Claim 27, wherein said composition is adapted for administration to a knee joint.

35. A method according to Claim 34, wherein said composition comprises at least 10 mg of said NF-κB decoy.

36. A method according to Claim 34, wherein said composition comprises at least 30 mg of said NF-κB decoy.

37. Use of at least one NF-κB decoy in manufacturing a medicament for treating and preventing an inflammatory disease or disorder.

38. Use of at least one NF-κB decoy in manufacturing a medicament for treating and preventing an articular disease or disorder.

## Fig.1  Changes observed in simian collagen arthritis models ( 14 weeks )

**No treatment**                **Scrambled decoy**                **NF-κB decoy**

## Fig.2 Changes observed in the rat osteoarthritis models ( 7 weeks )

Normal Donor

Collagen -derived arthritis model

NF-κB decoy (10mg)　　　Steroid treatment　　　NF-κB decoy (30mg)

**Fig.3 Administration schedule**

**Elapsed time ( weeks )**

| 0 | 1W | 3W | 5W | 7W | 9W | 11W | 13W |

Sensitiza·tion

Administra-tion group (two-week intervals)

Administration group (four-week intervals)

Steroid Administration group (two-week intervals)

Collection of blood sample

X-ray

Autopsy

Bone mass measurement

# Fig.4 Administration schedule

## Elapsed time ( weeks )

0  1W  2W  3W  4W  5W  6W  7W  8W  9W

Operation

Administra-
tion (one-week
intervals)

n=3

FITC-NF-κB
administration

n=3

Collection of
blood sample
X-ray
Autopsy

EP 1 512 415 A1

## INTERNATIONAL SEARCH REPORT

International application No.

PCT/JP03/06299

A. CLASSIFICATION OF SUBJECT MATTER
Int.Cl[7] A61K48/00, 45/00, 31/7088, 9/127, A61P1/16, 13/02, 13/08,
13/10, 13/12, 19/02, 29/00

According to International Patent Classification (IPC) or to both national classification and IPC

B. FIELDS SEARCHED

Minimum documentation searched (classification system followed by classification symbols)
Int.Cl[7] A61K48/00, 45/00, 31/7088, 9/127, A61P1/16, 13/02, 13/08,
13/10, 13/12, 19/02, 29/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)
CAPLUS(STN), REGISTRY(STN), BIOSIS(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X | TOMITA, Tetsuya et al., Suppressed severity of collagen-induced arthritis by in vivo transfection of unclear factor κB decoy oligodeoxynucleotides as a gene therapy, ARTHEITIS & RHEUMATISM, 1999, Vol.42, No.12, pages 2532 to 2542; full text; particularly, page 2532, left column | 1-18,37,38 |
| X | TOMITA, T. et al., Transcription factor decoy for NF κB inhibits cytokine and adhension molecule expressions in synovial cells derived from rheumatoid arthritis, Rheumatology, 2000, Vol.39, No.7, pages 749 to 757, full text; particularly, page 749, abstract | 1-18,37,38 |

☒ Further documents are listed in the continuation of Box C.       ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 July, 2003 (23.07.03) | 19 August, 2003 (19.08.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

International application No.

PCT/JP03/06299

| C (Continuation). DOCUMENTS CONSIDERED TO BE RELEVANT | | |
|---|---|---|
| Category* | Citation of document, with indication, where appropriate, of the relevant passages | Relevant to claim No. |
| X | MIAGKOV, Alexei V. et al., NF-κB activation provides the potential link between inflammation and hyperplasia in the arthritic joint, Proc.Natl. Acad.Sci.U.S.A., 1998, Vol.95, No.23, pages 13859 to 13864, full text; particularly, page 13859, abstract; Fig. 1 | 1-18,37,38 |
| X | EP 824918 A1 (FUJISAWA PHARMACEUTICAL CO., LTD.), 25 February, 1998 (25.02.98), Full text; particularly, Claim 2; page 4, lines 19 to 20 & WO 96/35430 A1 | 1-18,37,38 |

Form PCT/ISA/210 (continuation of second sheet) (July 1998)

## INTERNATIONAL SEARCH REPORT

| International application No. |
| --- |
| PCT/JP03/06299 |

**Box I   Observations where certain claims were found unsearchable (Continuation of item 2 of first sheet)**

This international search report has not been established in respect of certain claims under Article 17(2)(a) for the following reasons:

1. ☒ Claims Nos.: 19-36

   because they relate to subject matter not required to be searched by this Authority, namely:

   Claims 19 to 36 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II   Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest.

☐ No protest accompanied the payment of additional search fees.

Form PCT/ISA/210 (continuation of first sheet (1)) (July 1998)

SEQUENCE LISTING

<110> Anges MG, Inc.

<120>  NFkappaB decoy composition for treating brain ischemic disorders

<130>  P021775EP

<150>  JP2002-156524

<151>  2002-05-29

<160>  8

<170>  PatentIn version 3.0

<210>  1
<211>  20
<212>  DNA
<213>  Artificial

<220>
<223>  NFKappaB decoy
<400>  1
ccttgaaggg atttccctcc                                                        20

<210>  2
<211>  28
<212>  DNA
<213>  Artificial

<220>
<223>  STAT-1 decoy
<400>  2

gatctaggga tttccgggaa atgaagct                                                   28

<210> 3

<211> 16

<212> DNA

<213> Artificial

<220>

<223> GATA-3 decoy

<400> 3
agcttgagat agagct                                                                16

<210> 4

<211> 28

<212> DNA

<213> Artificial

<220>

<223> STAT-6 decoy

<400> 4
gatcaagacc ttttcccaag aaatctat                                                   28

<210> 5

<211> 19

<212> DNA

<213> Artificial

<220>

<223>  Ets decoy

<400> 5
agcttgtgag tcagaagct                                                             19

<210> 6

<211> 20

<212> DNA

```
<213>  Artificial

<220>

<223>   Ets decoy

<400>  6
aattcaccgg aagtattcga                                              20

<210>  7

<211>  20

<212>  DNA

<213>  Artificial

<220>

<223>  NFkappa B decoy

<400>  7
catgtcgtca ctgcgctcat                                              20

<210>  8

<211>  20

<212>  DNA

<213>  Artificial

<220>

<223>  scrambled decoy ODN

<400>  8
atgagcgcag tgacgacatg                                              20
```